# FASCICULE DE BREVET EUROPEEN

(11) **EP 3 210 022 B1**
(45) Date de publication et mention de la délivrance du brevet: **13.05.2020**
(21) Numéro de dépôt: 15786909.0
(22) Date de dépôt: 23.10.2015
(51) Int. Cl.: G01N 33/543, G01N 33/569

(54) **PROCÉDÉ DE DÉTECTION D'ORGANISMES DANS UN ÉCHANTILLON DILUÉ**
VERFAHREN ZUM NACHWEIS VON ORGANISMEN IN EINER VERDÜNNTEN PROBE
METHOD FOR DETECTING ORGANISMS IN A DILUTED SAMPLE

(30) Priorité: 24.10.2014 FR 1460246
(43) Date de publication de la demande: 30.08.2017
(73) Titulaire: Viovy, Jean-Louis, 75013 Paris (FR); Descroix, Stéphanie, 75014 Paris (FR); Malaquin, Laurent, 31450 Aiguesvives (FR); Pereiro, Iago, 8003 Zürich (CH); Alexandre, Lucile, 75014 Paris (FR)
(72) Inventeur: Viovy, Jean-Louis, 75013 Paris (FR); Descroix, Stéphanie, 75014 Paris (FR); Malaquin, Laurent, 31450 Aiguesvives (FR); Pereiro, Iago, 8003 Zürich (CH); Alexandre, Lucile, 75014 Paris (FR)
(74) Mandataire: Bandpay & Greuter
(86) Numéro de dépôt international: PCT/EP2015/074654
(87) Numéro de publication internationale: WO 2016/062878

(56) Documents cités:
- WO-A1-2013/121216
- WO-A1-2014/037674
- WO-A2-2006/066216
- WO-A2-2013/126774
- WO-A2-2013/165615
- TAE SEOK SEO: "Highly integrated microdevice for ultrasensitive pathogen detection", NANO/MICRO ENGINEERED AND MOLECULAR SYSTEMS (NEMS), 2010 5TH IEEE INTERNATIONAL CONFERENCE ON, IEEE, PISCATAWAY, NJ, USA, 20 janvier 2010 (2010-01-20), pages 1065-1068, XP031768933, ISBN: 978-1-4244-6543-9
- NATHANIEL BEYOR ET AL: "Integrated Capture, Concentration, Polymerase Chain Reaction, and Capillary Electrophoretic Analysis of Pathogens on a Chip", ANALYTICAL CHEMISTRY, vol. 81, no. 9, 1 mai 2009 (2009-05-01), pages 3523-3528, XP055232362, US ISSN: 0003-2700, DOI: 10.1021/ac900060r

## Description

### DOMAINE DE L'INVENTION

La présente invention concerne un procédé de détection d'organismes dans un échantillon dilué. Elle concerne également un procédé de préparation d'organismes à partir d'un échantillon dilué. Elle concerne encore des systèmes fluidiques adaptés à la mise ens œuvre de ces procédés.

### ARRIERE-PLAN TECHNIQUE

La détection, la caractérisation, l'identification d'espèces biologiques et en particulier d'organismes tels que des bactéries est un besoin qui concerne de nombreux domaines, par exemple le diagnostic médical ou vétérinaire, la sécurité alimentaire ou l'environnement. C'est aussi un besoin dans différents domaines de la production, en biotechnologie et en pharmaceutique. C'est enfin un besoin dans de nombreux domaines de la recherche en sciences de la vie ou en pharmacie.

Un exemple particulièrement important est celui des bactéries. Si certaines sont utilisées dans l'industrie pour la production d'aliments, de produits chimiques ou de médicaments, mais aussi pour le traitement des eaux usées, d'autres peuvent se révéler dangereuses pour la santé. Dans de nombreux cas, la rapidité de la détection et de l'identification de ces bactéries est donc critique. Par ailleurs, il n'est souvent pas suffisant de détecter des traces de bactéries, mais il est souvent également nécessaire de connaître leur état (vivantes ou mortes), leur infectiosité ou leur résistance à tel ou tel antibiotique.

Par conséquent, il est souhaitable de savoir détecter des bactéries dans un échantillon contenant éventuellement une grande variété de microorganismes. Le temps de réponse doit être le plus court possible, le taux de confiance le plus élevé possible, et les manipulations à effectuer les plus simples possibles.

Actuellement, le système standard d'identification des bactéries passe par la culture de colonies sur un milieu plus ou moins sélectif selon les cas, et le comptage manuel, ou l'analyse après culture du contenu des colonies, par exemple par des technologies d'analyse moléculaire comme la spectrométrie de masse. Il faut alors attendre approximativement 24 à 48 heures pour le développement des bactéries. De plus, les installations nécessaires, ainsi que le personnel, doivent être spécialisés, même si les outils de programmation informatique peuvent être utiles. Pour identifier les souches de bactéries, il faut en outre effectuer une comparaison morphologique, des tests de résistance aux antibiotiques, ou encore une identification par méthode colorimétrique, ou une combinaison de telles caractérisations. Cette méthode est donc peu chère, assez fiable et facile à mettre en œuvre, mais elle demande une concentration importante en bactéries, ainsi qu'un temps d'opération très important.

Dans le document CN 102094062, il a été proposé une méthode dans laquelle l'échantillon est passé à travers un filtre, afin que les bactéries s'accumulent dans le filtre ; ces bactéries sont ensuite cultivées en plaçant le filtre dans un milieu de culture, et les métabolites provenant de la croissance des bactéries sont détectés. Cette méthode est complexe et nécessite un temps d'analyse long.

Il est également connu de cultiver des cellules dans des systèmes à base de lits fluidisés. Par exemple, il a été proposé dans le document CN 201933088 un système pour la culture de cellules de mammifères adhérentes sur un substrat solide, comprenant un lit fluidisé compris entre deux plaques poreuses, et perfusé par un gaz pour favoriser la culture. Ce système n'est toutefois pas adapté à des applications de diagnostic ou de détection sur des échantillons de faible volume.

Dans le document CN 1303924, il a été proposé une méthode pour produire des virus à l'intérieur de cellules animales, elles-mêmes adhérentes à des particules maintenues au sein d'un lit fluidisé stabilisé magnétiquement. La méthode prévoit une oxygénation, l'échange de milieu de culture, et la collection des virus en sortie de chambre. Cette méthode est adaptée à la production ; elle requiert l'injection préalable d'un grand nombre de cellules et ne permet donc pas la détection de cellules rares.

Il a été proposé dans le document WO 86/05202 encore un autre système de culture cellulaire sur lit fluidisé, pour la culture de tissus ou la fermentation, impliquant le traitement d'une partie du fluide traité dans le lit fluidisé dans une chambre annexe, et sa réinjection dans le lit. Ce système requiert encore un grand nombre initial de cellules, et il n'est donc pas adapté à la détection de cellules rares.

D'autres travaux se sont intéressés à des méthodes de détection plus directes et sensibles, dans des dispositifs microfluidiques.

Par exemple, dans l'article Soft inertial microfluidics for high throughput separation of bacteria of bacteria from human blood cells, in Lab Chip, 9:1193-1199 (2009), une séparation continue de cellules par méthode physique, en utilisant la relation entre l'inertie des objets à trier et leur dimensions caractéristiques, a été décrite. Cette méthode permet de travailler à fort débit, mais la séparation reste imprécise en termes de résolution en taille. Par ailleurs, beaucoup de bactéries ou cellules différentes peuvent avoir la même taille, et ce critère de tri est donc insuffisant.

Des méthodes de tri électrophorétiques peuvent également être employées. En effet, la mobilité électrophorétique d'une bactérie dépend de la charge et du rayon de la bactérie. L'électrophorèse permet ainsi de concentrer les bactéries recherchées, mais aussi d'écarter les bactéries mortes, en modifiant sur des temps très courts le sens électrique du champ appliqué, comme décrit dans l'article Enrichement of viable bacteria in a micro-volume by free-flow electrophoresis, in Lab Chip, 12:451-457 (2012). Alternativement, la force de diélectrophorèse qui repose sur les différences de constantes diélectriques des objets à trier, peut être employée.

De telles méthodes doivent être couplées à des systèmes de PCR (réaction de polymérisation en chaîne) ou à d'autres analyses génomiques afin d'identifier les bactéries en présence, comme exposé dans l'article A microfluidic chip intergrating DNA extraction and real-time PCR for the detection of bacteria in saliva, in Lab Chip, 13:1325-1332 (2013). Cependant, la spécificité et la sensibilité restent souvent insuffisantes.

Encore une autre méthode est celle de la capture par liaison entre anticorps et antigène. On peut notamment citer une utilisation de cette méthode pour une capture de bactéries par greffage d'anticorps directement sur les canaux microfluidiques d'un circuit, dans l'article On-chip microfluidic biosensor for bacterial detection and identification, in Sensors and Actuators B, 126:508-514 (2007). Dans ce cas, le greffage des anticorps s'effectue par silanisation, et il est nécessaire de laisser sédimenter les bactéries pour que la capture soit efficace. Le débit est insuffisant quand il s'agit de repérer quelques bactéries dans des échantillons de grand volume, comme c'est le cas par exemple pour les analyses diagnostiques et de sécurité alimentaire.

Dans l'article Immunomagnetic bead-based cell concentration microdevice for dilute pathogen detection, in Biomed Microdevices, 10:909-917 (2008), un système microfluidique est décrit dans lequel des billes magnétiques porteuses d'anticorps sont immobilisées par un aimant latéral, pour capturer des bactéries. Cependant l'efficacité de capture n'est que de 2 UFC par µL, ce qui est très insuffisant pour les taux d'efficacité demandés, par exemple dans les applications de diagnostic ou d'analyse alimentaire.

Dans le document WO 2014/037674 un lit fluidisé microfluidique est décrit, avec une circulation de billes magnétiques contrôlée par un champ magnétique externe, notamment pour la capture de bactéries, toujours par greffage d'anticorps spécifiques sur les billes magnétiques. Cependant, ce système ne dispose pas de la sensibilité suffisante pour détecter quelques bactéries dans de grands volumes d'échantillons. Par ailleurs, il ne permet pas de différencier des bactéries vivantes de bactéries mortes.

Il existe également des types de cellules particulièrement intéressantes à caractériser, concentrer, cultiver et/ou amplifier : il s'agit par exemple de levures, ou de cellules d'eucaryotes supérieurs, notamment de cellules mammifères et particulièrement de cellules humaines. Par exemple la biotechnologie et la médecine, notamment la médecine régénératrice ou le médecine des greffes, s'intéressent à de nouveaux procédés de caractérisation, de tri et de développement de cellules souches, de cellules progénitrices, de cellules pluripotentes ou totipotentes. Par exemple, dans l'article Microfluidic Capture of Endothelial Colony-Forming Cells from Human Adult Peripheral Blood: Phenotypic and Functional Validation In Vivo, in Tissue Engineering Part C, vol. 21:274-283 (2015), doi:10.1089/ten.tec.2014.0323., Lin *et al.* décrivent un procédé permettant la capture microfluidique de cellules humaines, des cellules endothéliales formatrices de colonies (ECFC). Cependant, dans ce procédé, la microfluidique ne sert qu'à la capture, et il est ensuite nécessaire de reporter les cellules d'intérêt dans des plaques de culture conventionnelles, ce qui conduit à un procédé long et coûteux en temps de manipulation.

Le document WO 2006/066216 décrit une méthode de détection de microorganismes dans un échantillon, comprenant la mise en contact de l'échantillon avec un module de concentration à biocapteur, afin de permettre la croissance des microorganismes ainsi que la détection de la croissance des microorganismes.

Le document WO 2013/165615 décrit des procédés et dispositifs pour la détection et/ou l'identification de microorganismes spécifiques dans un échantillon de culture. Des particules indicatrices peuvent former un complexe avec les microorganismes spécifiques dans l'échantillon. Des particules magnétiques peuvent être utilisées pour capturer ce complexe et le concentrer dans un emplacement localisé d'un récipient de test pour détection et identification.

Le document WO 2013/126774 décrit des procédés et dispositifs microfluidiques pour la capture, la détection, la culture, l'incubation ou l'analyse d'une espèce cible dans un fluide biologique. Des particules magnétiques spécifiques de l'espèce cible peuvent être utilisées pour faciliter la séparation de l'espèce cible du fluide et/ou pour récupérer l'espèce cible séparée à partir des dispositifs microfluidiques.

Le document WO 2013/121216 décrit un procédé de capture d'une cible cellulaire à partir d'une suspension, comprenant la fourniture d'une surface de liaison à laquelle un agent de liaison spécifique capable de lier la cible cellulaire est fixé de manière non-covalente, la formation d'un complexe dans la suspension entre la cible cellulaire et un agent de liaison spécifique, la mise en contact de la surface de liaison avec la suspension contenant le complexe, et l'incubation de sorte à capturer la cible cellulaire sur la surface de liaison.

Le document Highly Integrated Microdevice for Ultrasensitive Pathogen Detection, de Tae Seok Seo (Proc. 2010 5th IEEE International Conférence on Nano/Micro Engineered and Molecular Systems, p.1065-1068) décrit un système de laboratoire sur puce qui intègre une capture de cellules, une préconcentration, une réaction de polymérisation en chaîne (PCR) et une électrophorèse capillaire, pour la détection d'agents pathogènes.

Le document Integrated Capture, Concentration, Polymerase Chain Reaction and Capillary Electrophoretic Analysis of Pathogens on a Chip, de Nathaniel Beyor et al. (Anal. Chem. vol.81, n°9, p.3523-3528, 2009) décrit un système microfluidique qui permet la capture et la préconcentration de cellules sous la forme d'un lit fluidisé suivies par une réaction de PCR ainsi que par une électrophorèse capillaire pour la détection d'agents pathogènes.

Il existe donc un réel besoin de développer des systèmes pour repérer des bactéries rares dans des volumes d'échantillons assez importants, de façon facile, automatisée et rapide, et d'identifier leur infectiosité ou leur pouvoir de prolifération. Des problèmes similaires existent également pour d'autres organismes unicellulaires ou pluricellulaires, comme des levures ou des parasites, ou encore des cellules eucaryotes, par exemple des cellules souches ou encore des cellules cancéreuses.

### RESUME DE L'INVENTION

La présente invention permet de surmonter les inconvénients de l'état de la technique et a été définie dans les revendications. Elle fournit notamment un procédé tel que défini dans la revendication 1 de détection d'organismes (tels que des bactéries) dans un échantillon de liquide par capture d'un nombre même très faible d'organismes sur des particules retenues dans une zone de capture, puis multiplication ou croissance de ces organismes *in situ* dans la zone de capture, et enfin détection des organismes ainsi multipliés.

L'utilisation d'un lit fluidisé de particules dans le procédé permet d'utiliser des débits de fluides relativement importants, et d'optimiser les interactions entre les différentes espèces en jeu dans la zone de capture (et notamment entre les particules et les organismes susceptibles de s'y lier).

Ainsi, la divulgation concerne les objets suivants.

Objet 1. Procédé de détection d'organismes dans un échantillon liquide, comprenant la fourniture d'une zone de capture comportant des particules en milieu liquide et soumise à un flux hydrodynamique, les organismes à détecter étant susceptibles de se lier à ces particules, le procédé comprenant les étapes :
(a) de circulation de l'échantillon à travers la zone de capture ;
(b) de circulation d'un milieu de croissance à travers la zone de capture ; et
(c) de détermination de la présence, de la nature ou de la concentration des organismes dans la zone de capture ;
lesdites particules étant retenues dans la zone de capture sous la forme d'un lit fluidisé pendant au moins une partie de ces étapes.

Le terme « *organisme* » dans la présente demande désigne tout microorganisme ou tout organisme unicellulaire ou pluricellulaire susceptible de croître dans un milieu approprié, appelé ici « *milieu de croissance* ».

Le terme s'applique également aux organismes sous forme quiescente, par exemple des spores, des coques, etc. Le terme englobe également les cellules individuelles ou les agrégats cellulaires issus d'organismes plus complexes, comme par exemple les cellules de mammifères, naturelles ou mutées.

Le terme de « *lit fluidisé* » dans la présente demande désigne un état dans lequel les particules en suspension dans un milieu liquide en écoulement se comportent comme un fluide, c'est-à-dire qu'elles, sont en mouvement les unes par rapport aux autres, et peuvent adapter leur configuration mutuelle à la forme de la chambre dans lequel elles sont contenues, mais ne sont pas globalement entraînées par ledit fluide, comme cela sera exposé plus en détail ci-dessous.

Le terme de « *lit fluidisé* » est défini par opposition à un lit non-fluidisé, également appelé « *lit compact* », mais aussi par opposition à une simple suspension, dans laquelle les particules sont globalement entraînées à la vitesse du fluide.

L'invention prévoit ainsi une croissance (par exemple multiplication) des organismes cibles lors de l'étape (b) de culture des organismes *in situ* dans la zone de capture. Ainsi, la détection est facilitée, et la sensibilité est augmentée.

D'autre part, l'invention permet de différencier des organismes vivants d'organismes morts ; ou de différencier des organismes capables de proliférer d'organismes non capables de proliférer ou de se développer ; ou de différencier des organismes capables de proliférer ou de se développer plus ou moins vite.

Ainsi, l'invention permet d'obtenir des informations sur le métabolisme, la capacité de prolifération ou d'autres propriétés de certains organismes dans certains environnements.

Le milieu de croissance peut comprendre des nutriments pour les organismes.

Les particules peuvent être retenues dans la zone de capture sous la forme d'un lit fluidisé pendant une partie, et de préférence pendant la totalité, de l'étape (a).

Les particules peuvent être retenues dans la zone de capture sous la forme d'un lit fluidisé pendant une partie, et de préférence pendant la totalité, de l'étape (b).

Les particules peuvent être retenues dans la zone de capture sous la forme d'un lit fluidisé pendant une partie, et de préférence pendant la totalité, de l'étape (c).

Les particules peuvent être retenues dans la zone de capture sous la forme d'un lit compact pendant une partie, et de préférence pendant la totalité, de l'étape (c).

La vitesse du flux hydrodynamique peut être décroissante dans la zone de capture, dans la direction du flux hydrodynamique.

La retenue des particules dans la zone de capture est obtenue par application d'une force s'opposant au flux hydrodynamique.

La force s'opposant au flux hydrodynamique peut être décroissante dans la zone de capture, dans la direction du flux hydrodynamique.

La force s'opposant au flux hydrodynamique peut être essentiellement alignée avec la direction du flux hydrodynamique.

La force s'opposant au flux hydrodynamique peut être une force magnétique, les particules étant des particules magnétiques, de préférence super-paramagnétiques.

La force s'opposant au flux hydrodynamique peut être une force électrostatique, les particules étant des particules chargées.

La force s'opposant au flux hydrodynamique peut être une force de gravité ou centrifuge, les particules ayant une densité différente de celle d'un liquide environnant.

Les organismes à détecter peuvent être susceptibles de se lier aux particules par l'intermédiaire de ligands, de préférence des anticorps.

Les particules peuvent présenter une taille moyenne Dv50 de 1 nm à 500 µm, de préférence de 50 nm à 100 µm, et plus particulièrement de 1 µm à 50 µm.

Les organismes peuvent être choisis parmi les cellules procaryotes, les bactéries, les levures, les parasites unicellulaires ou pluricellulaires, les cellules fongiques, les cellules eucaryotes et notamment les cellules de mammifères ou les cellules de plantes, ou encore des ensembles fonctionnels de telles cellules ; selon certains modes préférés, lesdits organismes sont choisis parmi les organismes pathogènes, les cellules souches, les organismes génétiquement modifiés, et les cellules cancéreuses.

Les organismes peuvent être choisis parmi les cellules endothéliales, les cellules hématopoïétiques, les cellules épithéliales, les cellules fœtales, les cellules pluripotentes, les cellules totipotentes non-humaines, et les cellules souches pluripotentes induites.

L'étape (c) peut être effectuée par la mesure d'une propriété dans la zone de capture.

L'étape (c) peut être effectuée par la mesure d'une propriété hors de la zone de capture.

L'étape (c) peut être effectuée au cours de l'étape (b), de manière ponctuelle ou répétée au cours du temps.

L'étape (c) peut être effectuée par la détection, la culture ou l'identification d'organismes ou d'espèces transportés hors de de la zone de capture par flux hydrodynamique au cours de l'étape (b).

L'étape (c) peut être effectuée après l'étape (b).

Le procédé peut comprendre :
- le transfert d'espèces hors de la zone de capture après l'étape (b), l'étape (c) étant effectuée par mesure d'une propriété sur les espèces transférées ; les espèces étant de préférence des espèces sécrétées par les organismes éventuellement fixés aux particules dans la zone de capture, ou des constituants de ces organismes, le cas échéant libérés par une lyse.

Le procédé peut comprendre :
- le prélèvement de particules hors de la zone de capture après l'étape (b), l'étape (c) étant effectuée par mesure d'une propriété sur les particules prélevées.

Le procédé peut comprendre en outre :
- le transfert d'espèces hors de la zone de capture après l'étape (b) ou durant l'étape (b).

L'étape (c) peut être effectuée par mesure d'une propriété sur les espèces transférées.

Les espèces peuvent être sécrétées par des organismes liés aux particules dans la zone de capture, ou des constituants de ces organismes, le cas échéant libérés par une lyse.

Les espèces peuvent être des organismes liés aux particules dans la zone de capture, ou des organismes issus de tels organismes.

La propriété mesurée peut être une propriété biologique ou biochimique, telle qu'une affinité, une capacité de prolifération, un phénotype ou une propriété phénotypique, un génotype ou un caractère génétique, une mutation, un taux d'expression, une morphologie ou une capacité de prolifération.

Plus généralement, par « *propriété mesurée* », on entend toute caractéristique permettant de différencier une molécule d'autres molécules, un assemblage moléculaire d'autres assemblages moléculaires, ou une cellule d'autres cellules, ou un type cellulaire d'autres types cellulaires, ou un état d'une cellule d'autres états d'une cellule, un organisme d'autres organismes.

La propriété mesurée peut être une force, un déplacement, une pression, un débit, une masse, une densité, une porosité, une viscosité, une élasticité, une viscoélasticité, une densité optique, une turbidité, une propriété de texture, une mesure d'intensité ou un coefficient d'absorption de rayonnement.

La propriété mesurée peut être le volume occupé par les particules dans la zone de capture ou une dimension de ce volume occupé, optionnellement lorsque les particules sont sous la forme d'un lit compact.

L'expression « *volume occupé parles particules* » s'entend en général comme désignant le volume de la zone de capture dans lequel les particules (et de préférence les organismes) sont localisées, ou volume du lit de particules.

La concentration d'organismes dans l'échantillon peut être déterminée en fonction du temps nécessaire au cours de l'étape (b) pour que le volume occupé par les particules dans la zone de capture atteigne une valeur seuil.

L'étape (c) peut comporter une analyse biologique ou biochimique, de préférence une analyse génétique, une analyse protéomique, une analyse de toxines, ou une mesure d'activité métabolique, et de manière plus particulièrement préférée par amplification et/ou hybridation d'acide nucléique, séquençage d'acide nucléique, immunoagglutination ou dosage d'immunoadsorption par enzyme liée.

L'étape (b) peut permettre d'augmenter le nombre d'organismes dans la zone de capture d'un facteur au moins 10, de façon préférée au moins 100, voire au moins 1.000 ou au moins 10.000 ou au moins 100.000, par rapport au nombre d'organismes présents dans la zone de capture à la fin de l'étape (a).

Le procédé peut être mis en œuvre dans un système microfluidique.

Le procédé peut comporter une amplification moléculaire avant l'étape (c) ou au cours de l'étape (c).

Le procédé peut comprendre en outre la circulation d'un milieu comprenant un agent cible pour les organismes à travers la zone de capture, de préférence : soit après l'étape de circulation du milieu de croissance ; soit ou au cours de ladite étape, l'agent cible étant de manière plus particulièrement préférée incorporé au milieu de croissance.

Le procédé peut être pour le criblage de médicaments ou biocides, dans lequel l'agent cible est un médicament ou un biocide potentiel.

L'échantillon peut être issu de l'environnement, ou d'un produit alimentaire, ou d'un fluide corporel.

On entend « *environnement* » dans son sens le plus large à savoir tout type de support solide, gazeux ou liquide, et tout type d'application, tels que par exemple et de façon non limitative, de l'air de l'eau, des sols, des bâtiments, des installations humaines, des vêtements, des surfaces, des plantes, des ustensiles, des déchets, ou des éléments en lien avec la sécurité, les analyses forensiques.

De même, on entend « *fluide corporel* » dans son sens le plus large, comprenant par exemple du sang ou du sang traité, du plasma, du sérum, des larmes, du lait, de la salive, de la sueur, des échantillons provenant de prélèvement par écouvillons sur toutes parties du corps, de l'urine, des fèces, ou des échantillons fluides obtenus par tous types de prétraitements à partir de parties du corps, comme par exemple des biopsies ou des pièces opératoires. Les fluides corporels peuvent provenir de corps humains. Alternativement, ils peuvent provenir de tous types d'espèces animales ou végétales.

De même, la notion de « *produits alimentaires* » s'entend de la façon la plus large, qu'il s'agisse d'alimentation humaine ou animale, mais aussi de toute matière première, produit transformé, produit intermédiaire, effluent, entrant dans l'alimentation ou la production alimentaire.

Le procédé ci-dessus peut notamment être mis en œuvre grâce à un système fluidique de détection d'organismes dans un échantillon liquide, comprenant :
- au moins une chambre comportant au moins une entrée de fluide et une sortie de fluide et comportant une zone de capture ;
- des moyens de circulation de l'échantillon liquide à travers la zone de capture ;
- des moyens de circulation d'un milieu de croissance à travers la zone de capture ;
- des moyens d'application d'un champ de force dans la zone de capture, ledit champ de force étant configuré pour retenir des particules dans la zone de capture sous la forme d'un lit fluidisé lorsque la zone de capture est soumise à un flux hydrodynamique, les organismes à détecter étant susceptibles de se lier aux particules ;
- des moyens de détermination de la présence, de la nature ou de la concentration des organismes dans la zone de capture.

Le système fluidique peut comprendre au moins une chambre supplémentaire ou réservoir, en connexion fluidique avec une sortie de la chambre.

Le système fluidique peut comprendre en outre un dispositif de régulation de la température de la chambre.

Le système fluidique peut être un système microfluidique.

Dans le système fluidique, le champ de force peut être essentiellement aligné avec la direction du flux hydrodynamique dans la zone de capture.

Dans le système fluidique, le champ de force peut présenter une intensité décroissante dans le sens du flux hydrodynamique dans la zone de capture.

Dans le système fluidique, la chambre peut présenter une section orthogonale à la direction moyenne du flux hydrodynamique qui est croissante dans le sens du flux hydrodynamique, dans la zone de capture.

Dans le système fluidique, les moyens d'application d'un champ de force peuvent être des moyens d'application d'un champ magnétique.

Dans le système fluidique, les moyens d'application d'un champ de force peuvent être des moyens d'application d'un champ électrique.

Dans le système fluidique, les moyens de détermination peuvent comprendre des moyens de mesure d'une propriété dans la zone de capture.

Dans le système fluidique, les moyens de détermination peuvent comprendre des moyens de mesure d'une propriété dans une deuxième chambre en connexion fluidique avec la chambre comprenant la zone de capture.

Dans le système fluidique, les moyens de mesure de la propriété peuvent être des moyens de mesure de force, de déplacement, de pression, de débit, de masse, de densité, de porosité, de viscosité, d'élasticité, de viscoélasticité, de densité optique, de turbidité, de propriété de texture, d'intensité ou de coefficient d'absorption de rayonnement.

Dans le système fluidique, les moyens de mesure de la propriété peuvent être des moyens de mesure du volume occupé par les particules dans la zone de capture.

Le système fluidique peut comprendre des moyens d'analyse biologique ou biochimique, et de préférence des moyens d'analyse génétique, d'analyse protéomique ou de mesure d'activité métabolique, et de manière plus particulièrement préférée des moyens d'amplification et/ou d'hybridation d'acide nucléique, de séquençage d'acide nucléique, d'immunoagglutination ou de dosage d'immunoadsorption par enzyme liée.

Le système fluidique peut comporter des moyens d'amplification moléculaire, et les moyens de détermination peuvent être des moyens de détection de produits de l'amplification moléculaire.

Dans le système fluidique, la chambre comportant la zone de capture peut être dépourvue de filtre susceptible de retenir les particules.

La divulgation concerne aussi un programme d'ordinateur qui, lorsqu'il est exécuté, permet la mise en œuvre du procédé ci-dessus.

La divulgation concerne aussi un ensemble pour la détection d'organismes dans un échantillon liquide comprenant :
- le système fluidique ci-dessus ; et
- le programme d'ordinateur ci-dessus.

La divulgation concerne aussi un ensemble pour la détection d'organismes dans un échantillon liquide comprenant :
- le système fluidique ci-dessus ; et
- les particules auxquelles les organismes sont susceptibles de se lier.

La divulgation concerne aussi un ensemble pour la détection d'organismes dans un échantillon liquide comprenant :
- le système fluidique ci-dessus ; et
- le milieu de croissance.

La divulgation concerne aussi un ensemble pour la détection d'organismes dans un échantillon liquide comprenant :
- le système fluidique ci-dessus ;
- les particules auxquelles les organismes sont susceptibles de se lier ; et
- le milieu de croissance.

La divulgation concerne aussi un ensemble pour la détection d'organismes dans un échantillon liquide comprenant :
- le système fluidique ci-dessus ;
- le programme d'ordinateur ci-dessus ; et
- les particules auxquelles les organismes sont susceptibles de se lier.

La divulgation concerne aussi un ensemble pour la détection d'organismes dans un échantillon liquide comprenant :
- le système fluidique ci-dessus ;
- le programme d'ordinateur ci-dessus ;
- les particules auxquelles les organismes sont susceptibles de se lier ; et
- le milieu de croissance.

La divulgation concerne aussi les objets suivants, dans lesquels on ne prévoit pas nécessairement d'étape (b) permettant la croissance des organismes.

La divulgation concerne ainsi un procédé de détection d'organismes dans un échantillon liquide, comprenant la fourniture d'une zone de capture comportant des particules en milieu liquide et soumise à un flux hydrodynamique, les organismes à détecter étant susceptibles de se lier à ces particules, le procédé comprenant en outre les étapes :
- de circulation de l'échantillon à travers la zone de capture ;
- de mesure d'une propriété physique de la zone de capture, pendant et/ou après la circulation de l'échantillon, afin de déduire la présence ou la concentration ou la nature des organismes dans l'échantillon ;
les particules étant retenues dans la zone de capture sous la forme d'un lit fluidisé pendant au moins une partie de ces étapes.

Dans ce procédé, la propriété physique mesurée peut être choisie parmi les propriétés de viscosité, de viscoélasticité, de volume, de perméabilité et de densité.

Dans ce procédé, la propriété physique mesurée peut être une propriété physique des particules sous la forme d'un lit fluidisé.

Dans ce procédé, la propriété physique mesurée peut être une propriété physique des particules sous la forme d'un lit compact.

Ce procédé peut comprendre en outre une étape de circulation d'un milieu de croissance, de préférence comprenant des nutriments pour les organismes, pour les organismes à travers la zone de capture, l'étape de mesure étant effectuée simultanément et/ou postérieurement à cette étape de circulation d'un milieu de croissance.

La divulgation concerne aussi un procédé de préparation d'organismes à partir d'organismes parents présents dans un échantillon liquide, comprenant la fourniture d'une zone de capture comportant des particules en milieu liquide et soumise à un flux hydrodynamique, les organismes parents étant susceptibles de se lier à ces particules, le procédé comprenant les étapes :
(a) de circulation de l'échantillon à travers la zone de capture et de liaison des organismes parents aux particules ;
(b) de circulation d'un milieu de croissance à travers la zone de capture ; et
(c) de collecte, à la sortie de la zone de capture, d'organismes issus de la division des organismes parents ;
les particules étant retenues dans la zone de capture sous la forme d'un lit fluidisé pendant au moins une partie de ces étapes.

Dans ce procédé, l'étape (c) peut s'effectuer sans libération des organismes parents liés aux particules.

Dans ce procédé, l'étape (c) et l'étape (b) peuvent se superposer ou alterner au moins en partie.

Dans ce procédé, l'étape de circulation d'un milieu de croissance peut permettre d'augmenter le nombre d'organismes dans la zone de capture, ou le nombre d'organismes collectés à la sortie de la zone de capture, d'un facteur au moins 10, de façon préférée au moins 100, voire au moins 1.000 ou au moins 10.000 ou au moins 100.000, par rapport au nombre d'organismes présents dans l'échantillon initial.

Dans l'un quelconque des procédés ci-dessus, les particules peuvent être retenues dans la zone de capture sous la forme d'un lit fluidisé pendant une partie, et de préférence pendant la totalité, de l'étape de circulation de l'échantillon.

Le procédé peut être un procédé de détection tel que décrit ci-dessus, et dans lequel les particules sont retenues dans la zone de capture sous la forme d'un lit fluidisé pendant une partie, et de préférence pendant la totalité, de l'étape de mesure.

Le procédé peut être un procédé de détection tel que décrit ci-dessus, et dans lequel les particules sont retenues dans la zone de capture sous la forme d'un lit compact pendant une partie, et de préférence pendant la totalité, de l'étape de mesure.

Dans l'un quelconque des procédés ci-dessus, la vitesse du flux hydrodynamique peut être décroissante dans la zone de capture, dans la direction du flux hydrodynamique.

Dans l'un quelconque des procédés ci-dessus, la retenue des particules dans la zone de capture peut être obtenue par application d'une force s'opposant au flux hydrodynamique.

La force s'opposant au flux hydrodynamique peut être décroissante dans la zone de capture, dans la direction du flux hydrodynamique.

La force s'opposant au flux hydrodynamique peut être essentiellement alignée avec la direction du flux hydrodynamique.

La force s'opposant au flux hydrodynamique peut être une force magnétique, les particules étant des particules magnétiques, de préférence super-paramagnétiques.

La force s'opposant au flux hydrodynamique peut être une force électrostatique, les particules étant des particules chargées.

La force s'opposant au flux hydrodynamique peut être une force de gravité ou centrifuge, les particules ayant une densité différente de celle d'un liquide environnant.

Les organismes à détecter, respectivement les organismes parents, peuvent être susceptibles de se lier aux particules par l'intermédiaire de ligands, de préférence des anticorps.

Les particules peuvent présenter une taille moyenne Dv50 de 1 nm à 500 µm, de préférence de 50 nm à 100 µm, et plus particulièrement de 1 µm à 50 µm.

Les organismes peuvent être choisis parmi les bactéries, les parasites unicellulaires ou pluricellulaires, les champignons, les cellules eucaryotes et notamment les cellules de mammifères ou les cellules de plantes et, de préférence, les organismes sont choisis parmi les organismes pathogènes, les cellules souches et les cellules cancéreuses.

Les organismes peuvent être choisis parmi les cellules endothéliales, les cellules hématopoïétiques, les cellules épithéliales, les cellules fœtales, les cellules pluripotentes, les cellules totipotentes non humaines, et les cellules souches pluripotentes induites.

Lorsque le procédé est un procédé de détection tel que décrit ci-dessus, la propriété mesurée peut être le volume occupé par les particules dans la zone de capture ou une dimension de ce volume occupé.

L'un quelconque des procédés ci-dessus peut être mis en œuvre dans un système microfluidique.

L'un quelconque des procédés ci-dessus peut comprendre en outre une étape de circulation d'un milieu comprenant un agent cible pour les organismes à travers la zone de capture, optionnellement après l'étape de circulation du milieu de croissance.

L'un quelconque des procédés ci-dessus peut être pour le criblage de médicaments ou biocides, dans lequel l'agent cible est un médicament ou un biocide potentiel.

Dans l'un quelconque des procédés ci-dessus, l'échantillon peut être issu de l'environnement, ou d'un produit alimentaire, ou d'un fluide corporel.

La divulgation concerne également un système fluidique de détection d'organismes dans un échantillon liquide, comprenant :
- au moins une chambre comportant au moins une entrée de fluide et une sortie de fluide et comportant une zone de capture ;
- des moyens de circulation de l'échantillon liquide à travers la zone de capture ;
- des moyens d'application d'un champ de force dans la zone de capture, ledit champ de force étant configuré pour retenir des particules dans la zone de capture sous la forme d'un lit fluidisé lorsque la zone de capture est soumise à un flux hydrodynamique, les organismes à détecter étant susceptibles de se lier aux particules ;

- des moyens de mesure d'une propriété physique de la zone de capture ;
- des moyens de détermination de la présence ou de la concentration ou de la nature des organismes dans l'échantillon en fonction du résultat de cette mesure.

La divulgation concerne également un système fluidique de préparation d'organismes à partir d'organismes parents présents dans un échantillon liquide comprenant :
- au moins une chambre comportant au moins une entrée de fluide et une sortie de fluide et comportant une zone de capture ;
- des moyens de circulation de l'échantillon liquide à travers la zone de capture ;
- des moyens d'application d'un champ de force dans la zone de capture, ledit champ de force étant configuré pour retenir des particules dans la zone de capture sous la forme d'un lit fluidisé lorsque la zone de capture est soumise à un flux hydrodynamique, les organismes parents étant susceptibles de se lier aux particules ;
- des moyens de collecte, à la sortie de la zone de capture, d'organismes issus de la division des organismes parents.

Les moyens de détermination de la présence ou de la concentration ou de la nature des organismes présents dans l'échantillon peuvent comporter des moyens de collecte, de culture ou de multiplication d'organismes issus de la zone de capture.

Le système fluidique peut être un système microfluidique.

Dans l'un des systèmes fluidiques ci-dessus, le champ de force peut être essentiellement aligné avec la direction du flux hydrodynamique dans la zone de capture.

Dans l'un des systèmes fluidiques ci-dessus, le champ de force peut présenter une intensité décroissante dans le sens du flux hydrodynamique dans la zone de capture.

Dans l'un des systèmes fluidiques ci-dessus, la chambre peut présenter une section orthogonale à la direction moyenne du flux hydrodynamique qui est croissante dans le sens du flux hydrodynamique, dans la zone de capture.

Les systèmes fluidiques ci-dessus peuvent comprendre en outre des moyens de circulation d'un milieu de croissance à travers la zone de capture.

Dans les systèmes fluidiques ci-dessus, les moyens d'application d'un champ de force peuvent être des moyens d'application d'un champ magnétique.

Dans les systèmes fluidiques ci-dessus, les moyens d'application d'un champ de force peuvent être des moyens d'application d'un champ électrique.

Lorsque le système fluidique est un système fluidique de détection tel que décrit ci-dessus, les moyens de mesure de la propriété peuvent être des moyens de mesure de viscosité, de viscoélasticité, de volume, de perméabilité et de densité.

Lorsque le système fluidique est un système fluidique de détection tel que décrit ci-dessus, les moyens de mesure de la propriété peuvent être des moyens de mesure du volume occupé par les particules dans la zone de capture.

Dans l'un quelconque des systèmes fluidiques ci-dessus, la chambre comportant la zone de capture peut être dépourvue de filtre susceptible de retenir les particules.

La divulgation concerne aussi un programme d'ordinateur qui, lorsqu'il est exécuté, permet la mise en œuvre de l'un quelconque des procédés ci-dessus.

La divulgation concerne aussi un ensemble pour la détection d'organismes dans un échantillon liquide comprenant :
- le système fluidique ci-dessus ; et
- le programme d'ordinateur ci-dessus.

La divulgation concerne aussi un ensemble pour la détection d'organismes dans un échantillon liquide comprenant :
- le système fluidique ci-dessus ; et
- les particules auxquelles les organismes sont susceptibles de se lier.

La divulgation concerne aussi un ensemble pour la détection d'organismes dans un échantillon liquide comprenant :
- le système fluidique ci-dessus ; et
- un milieu de croissance.

La divulgation concerne aussi un ensemble pour la détection d'organismes dans un échantillon liquide comprenant :
- le système fluidique ci-dessus ;
- les particules auxquelles les organismes sont susceptibles de se lier ; et
- un milieu de croissance.

La divulgation concerne aussi un ensemble pour la détection d'organismes dans un échantillon liquide comprenant :
- le système fluidique ci-dessus ;
- le programme d'ordinateur ci-dessus ; et
- les particules auxquelles les organismes sont susceptibles de se lier.

La divulgation concerne aussi un ensemble pour la détection d'organismes dans un échantillon liquide comprenant :
- le système fluidique ci-dessus ;
- le programme d'ordinateur ci-dessus ;
- les particules auxquelles les organismes sont susceptibles de se lier ; et
- un milieu de croissance.

### BREVE DESCRIPTION DES FIGURES

La **figure 1** représente de manière schématique un mode de réalisation du système microfluidique selon l'invention.
Les **figures 2A à 2Q** représentent de manière schématique diverses formes possibles pour la chambre d'un système microfluidique selon l'invention.
La **figure 3** représente de manière schématique un détail d'un mode de réalisation du système microfluidique selon l'invention.
La **figure 4** est un graphe représentant la fluorescence moyenne d'un lit de particules ayant capturé des bactéries (en ordonnée, en unités arbitraires) en fonction de la durée d'incubation des particules avec un milieu de croissance, dans un système microfluidique de l'invention (en abscisse, en minutes). On se reportera à l'exemple 2 pour plus de détails.
La **figure 5** est un graphe représentant l'avancée du front du lit de particules ayant capturé des bactéries (en ordonnée, en mm) en fonction de la durée d'incubation des particules avec un milieu de croissance, dans un système microfluidique de l'invention (en abscisse, en minutes). Les différentes courbes correspondent à différentes concentrations initiales en bactéries dans l'échantillon de départ. On se reportera à l'exemple 3 pour plus de détails.
   - les particules auxquelles les organismes sont susceptibles de se lier ; et
   - un milieu de croissance.

Objet 91. Ensemble pour la détection d'organismes dans un échantillon liquide comprenant :
- le système fluidique selon l'un des objets 75 à 85 ;
- le programme d'ordinateur selon l'objet 86 ; et
- les particules auxquelles les organismes sont susceptibles de se lier.

Objet 92. Ensemble pour la détection d'organismes dans un échantillon liquide comprenant :
- le système fluidique selon l'un des objets 75 à 85 ;
- le programme d'ordinateur selon l'objet 86 ;
- les particules auxquelles les organismes sont susceptibles de se lier ; et
- un milieu de croissance.

### BREVE DESCRIPTION DES FIGURES

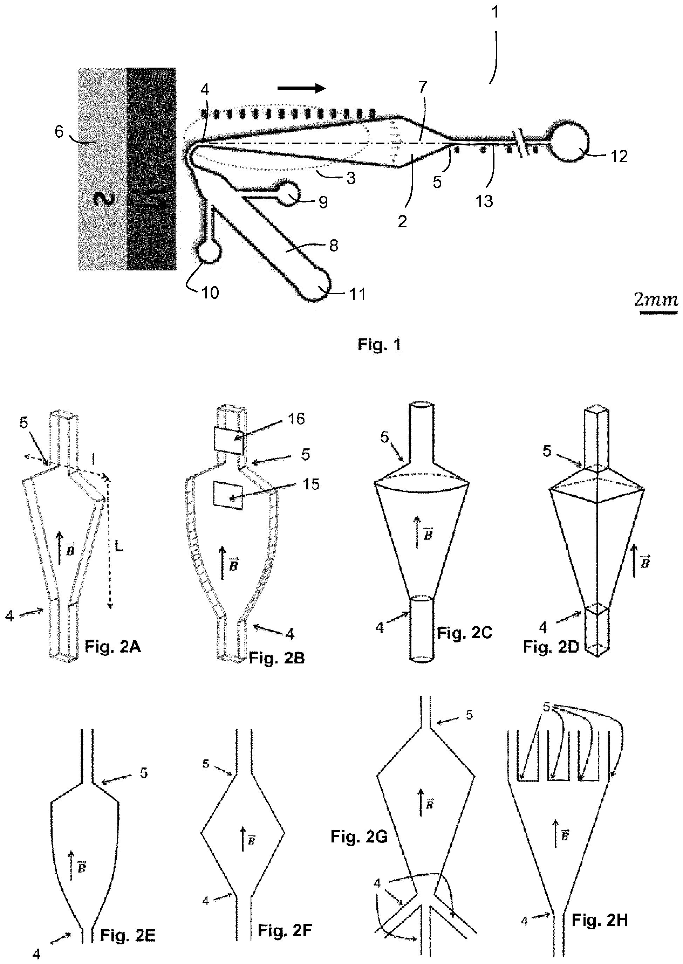

La **figure 1** représente de manière schématique un mode de réalisation du système microfluidique selon l'invention.
Les **figures 2A à 2Q** représentent de manière schématique diverses formes possibles pour la chambre d'un système microfluidique selon l'invention.

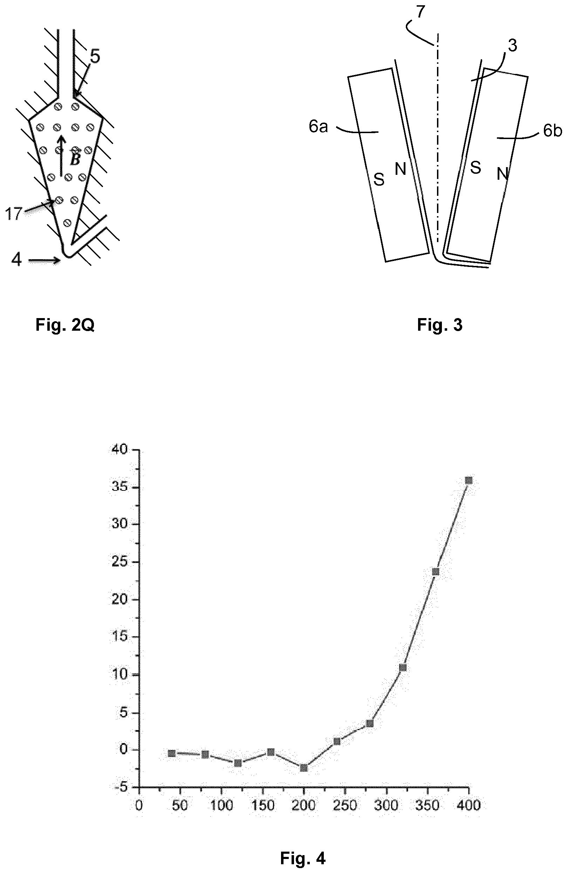

La **figure 3** représente de manière schématique un détail d'un mode de réalisation du système microfluidique selon l'invention.
La **figure 4** est un graphe représentant la fluorescence moyenne d'un lit de particules ayant capturé des bactéries (en ordonnée, en unités arbitraires) en fonction de la durée d'incubation des particules avec un milieu de croissance, dans un système microfluidique de l'invention (en abscisse, en minutes). On se reportera à l'exemple 2 pour plus de détails.

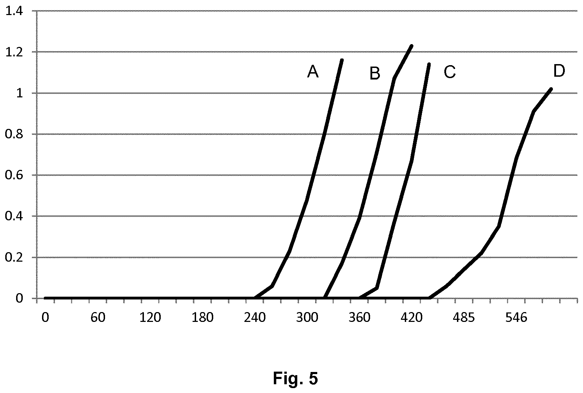

La **figure 5** est un graphe représentant l'avancée du front du lit de particules ayant capturé des bactéries (en ordonnée, en mm) en fonction de la durée d'incubation des particules avec un milieu de croissance, dans un système microfluidique de l'invention (en abscisse, en minutes). Les différentes courbes correspondent à différentes concentrations initiales en bactéries dans l'échantillon de départ. On se reportera à l'exemple 3 pour plus de détails.

### DESCRIPTION DE MODES DE REALISATION DE L'INVENTION

L'invention est maintenant décrite plus en détail et de façon non limitative dans la description qui suit.

### Zone de capture

L'invention repose sur la mise en œuvre d'une zone de capture dans une chambre d'un système fluidique, pour retenir des particules dans celle-ci tout en faisant circuler des fluides au travers de la zone de capture (de sorte à créer un écoulement dans la zone de capture, c'est-à-dire un flux hydrodynamique).

Ainsi, le système fluidique de l'invention comporte des moyens d'application d'un champ de force dans la zone de capture, ledit champ de force étant configuré pour retenir les particules dans la zone de capture.

Par simplification de langage, dans l'ensemble de la description, on entend par « *force s'opposant au flux hydrodynamique* » une force, ou une combinaison de forces, exercée sur au moins une particule, et s'opposant à la ou aux forces exercées sur cette particule par le flux hydrodynamique.

Le champ de force peut être électrique, diélectrique, électromagnétique, magnétique, gravitationnel (de pesanteur) ou centrifuge. De manière préférée, il est magnétique. Dans certains modes de mise en œuvre, les moyens d'application du champ de force sont associés à une forme particulière de la zone de capture.

Les moyens d'application peuvent notamment comprendre un système magnétique définissant dans la zone de capture un champ magnétique présentant un gradient, et capable de retenir les particules (magnétiques) dans la zone de capture.

Le système magnétique peut comprendre un ou plusieurs électroaimants, ou un ou plusieurs aimants permanents, ou une ou plusieurs pièces polaires, ou une combinaison de ceux-ci.

Le champ magnétique obtenu peut ainsi présenter dans une partie au moins de la zone de capture un gradient compris de 10 T/m à 10000 T/m, de préférence de 10 T/m à 5000 T/m, et de manière plus particulièrement préférée de 100 T/m à 1000 T/m.

Alternativement, le système fluidique divulgué peut comprendre un système électrique définissant dans la zone de capture un champ électrique capable de retenir les particules (chargées) dans la zone de capture.

Le système électrique peut en particulier comprendre au moins deux électrodes. Les électrodes peuvent par exemple être au contact de la zone de capture. Les électrodes peuvent également être au contact d'un liquide en connexion fluidique avec la zone de capture, dans des réservoirs annexes.

Alternativement, le système fluidique divulgué peut comprendre un système électrique définissant dans la zone de capture un champ électrique présentant un gradient capable de retenir des particules (diélectriques) dans la zone de capture.

Le système électrique peut en particulier comprendre une ou plusieurs électrodes. Les électrodes peuvent également être au contact de la zone de capture. Les électrodes peuvent également être au contact d'un liquide en connexion fluidique avec la zone de capture, dans des réservoirs annexes. Les électrodes peuvent également être situées au voisinage de la zone de capture, mais séparées de celle-ci par un isolant : cette variante est préférée lorsque le champ électrique présente une composante alternative.

Lorsqu'un système électrique est utilisé, il peut générer un champ électrique continu, alternatif, pulsé, ou une combinaison d'une composante continue et d'une composante non continue. Le champ électrique dans au moins une partie de la zone de capture (de préférence dans l'ensemble de la zone de capture) peut avoir notamment une amplitude de 1 à 10 V/cm, ou de 10 à 100 V/cm, ou encore de 100 à 1000 V/cm ; dans d'autres cas, et notamment en présence d'électrodes isolées de la chambre, ou lorsque le champ électrique présente un gradient, l'amplitude peut être de 1000 V/cm à 10000 V/cm, ou de 10000 à 100000 V/cm.

On peut également combiner entre eux les systèmes magnétiques et électriques décrits ci-dessus.

La zone de capture peut également être orientée dans le champ de pesanteur de telle façon que l'écoulement du fluide dans la zone de capture s'effectue du bas vers le haut. La retenue des particules peut alors être obtenue lorsque les particules présentent une densité supérieure à celle du fluide circulant.

Alternativement, la zone de capture peut être orientée dans le champ de pesanteur de telle façon que l'écoulement du fluide dans la zone de capture s'effectue du haut vers le bas. La retenue des particules peut alors être obtenue lorsque les particules présentent une densité inférieure à celle du fluide circulant.

Il est encore possible d'effectuer une rotation du dispositif, afin de générer au sein de la chambre une force centrifuge. La retenue des particules peut alors être obtenue lorsque le fluide circulant s'écoule dans un sens opposé à la force centrifuge et que les particules sont plus denses que le fluide circulant.

La force magnétique, électrique ou électrostatique, de pesanteur ou centrifuge, s'opposant à l'écoulement de fluide, a pour effet de retenir les particules dans la zone de capture.

Cette force peut être générée du fait, respectivement, d'une différence de susceptibilité magnétique ou électrique, ou d'une différence de conductivité, ou d'une différence de densité, entre les particules et le fluide environnant.

L'amplitude de la force s'opposant à l'écoulement peut comprendre une composante continue. Elle peut également comprendre une composante alternative.

La présence d'une composante alternative est préférée lorsque des particules diélectriques sont utilisées, ou lorsque la force est une force électrique produite par des électrodes isolées de la zone de capture, ou les deux.

La fréquence de la composante alternative peut par exemple valoir de 1 à 100 Hz ; ou de 100 Hz à 10 kHz ; ou de 10 kHz à 1 MHz ; ou de 1 à 100 MHz.

Le champ des forces s'opposant à l'écoulement peut être essentiellement stationnaire pendant une ou certaines étapes du procédé ou pendant l'ensemble du procédé ; c'est-à-dire que, en chaque point de la zone de capture, la direction et l'amplitude de la force ne varie essentiellement pas au cours du temps. Lorsque la force présente une composante alternative, l'absence de variation de direction et d'amplitude au cours du temps est évaluée sur une durée caractéristique d'au moins 100 fois la période de la composante alternative.

Le champ de forces peut également ne pas être stationnaire ; c'est-à-dire que, en certains points au moins de la zone de capture, la force varie en amplitude et/ou en direction au cours du procédé.

S'il s'agit d'une force magnétique, la variation peut être obtenue soit par variation d'un courant dans une bobine, soit par déplacement de milieux magnétiques ou magnétisables, par exemple par déplacement d'un aimant permanent, ou d'un ferrofluide, ou d'un shunt magnétique.

S'il s'agit d'une force électrique ou électrostatique, la variation peut être obtenue par changement d'un potentiel entre deux bornes d'un générateur.

S'il s'agit d'une force de pesanteur ou centrifuge, la variation peut être obtenue soit par une variation de l'inclinaison de la zone de capture par rapport à l'orientation du champ de pesanteur, soit par une variation d'une vitesse de rotation.

Le champ des forces s'opposant à l'écoulement peut présenter un gradient dans la zone de capture, c'est-à-dire que l'amplitude de la force peut varier en fonction de la position dans la zone de capture.

Le gradient de force dans la zone de capture peut être un gradient monotone, c'est-à-dire que l'amplitude de la force varie de façon monotone d'une extrémité à l'autre de la zone de capture. De manière préférée, l'amplitude de la force décroît dans au moins une partie de la zone de capture (de préférence dans toute la zone de capture) dans la direction de l'écoulement du fluide.

La direction de la force est de préférence la même (ou essentiellement la même) dans l'ensemble de la zone de capture. De manière plus particulièrement préférée, elle est essentiellement alignée avec la direction de l'écoulement du fluide.

Alternativement, la direction de la force peut être transverse par rapport à la direction de l'écoulement du fluide, ou avoir une orientation variable par rapport à la direction de l'écoulement du fluide, ou avoir une orientation variable selon la position dans la zone de capture.

Comme cela est décrit plus en détail ci-dessous, selon la géométrie de la chambre et la nature de la force utilisée pour retenir les particules, on peut obtenir une orientation générale de la force approximativement alignée avec celle de l'écoulement, sans que cet alignement soit nécessairement obtenu rigoureusement dans toute la chambre, d'une part à cause de la divergence du champ de force (par exemple la divergence du champ magnétique imposée par la forme des aimants utilisés), et d'autre part à cause de phénomènes d'écoulement inhérents aux lois de l'hydrodynamique, comme la présence de conditions de non-écoulement sur les bords de la chambre, et la présence d'un profil de Poiseuille. C'est pourquoi cette notion d'alignement s'entend de façon générale et non nécessairement absolue.

Par ailleurs, il peut être avantageux dans certains cas d'obtenir un champ de forces ayant une direction légèrement différente de celle de l'écoulement, afin de favoriser la recirculation de particules dans la chambre dans l'état de lit fluidisé.

### Lit fluidisé et lit compact

Selon un mode de réalisation, l'une au moins des étapes (a) à (c), et de préférence deux ou trois et de façon encore préférée toutes ces étapes, sont effectuées au moins en partie dans un régime dit de lit fluidisé, c'est-à-dire que les particules sont en mouvement les unes par rapport aux autres, tout en restant collectivement contenues dans la zone de capture et soumises à la circulation d'un fluide (en l'occurrence un liquide).

Le lit fluidisé est obtenu en soumettant les particules à une force s'opposant à l'écoulement hydrodynamique du fluide traversant la zone de capture.

Cette force est de préférence une force magnétique, mais peut alternativement être une force électrique, de gravité (ou pesanteur) ou centrifuge. Des combinaisons de ces forces sont également possibles.

Le lit fluidisé est de préférence un lit dense mais non compact. Ainsi, les particules se déplacent les unes relativement aux autres.

Un état compact est défini comme un état dans lequel les particules sont en contact permanent ou quasi-permanent avec les particules voisines. Un état dense mais non compact est un état dans lequel les particules entrent en contact les unes avec les autres occasionnellement, mais dans lequel les particules sont de manière prépondérante en contact avec le fluide, et non avec d'autres particules (la distance moyenne entre particules étant toutefois de préférence inférieure à 100 fois, ou à 50 fois, ou à 20 fois, ou à 15 fois, ou à 10 fois, ou à 5 fois, ou à 3 fois la taille moyenne des particules).

Ainsi, l'état dense mais non compact qui est obtenu dans le lit fluidisé correspond généralement, pour des particules sphériques ou approximativement sphériques, à une fraction volumique occupée par les particules de 0,01 à 0,3 dans la zone de capture, par exemple de 0,01 à 0,05 ou alternativement de 0,05 à 0,3.

Pour des particules non sphériques, et notamment pour des particules très allongées, l'état dense mais non compact qui est recherché dans le cadre de l'invention peut correspondre à une fraction volumique plus faible, par exemple de 0,001 à 0,1, puisque ces particules ont tendance à interagir plus facilement les unes avec les autres.

Selon un mode de réalisation, certaines des étapes du procédé de l'invention sont effectuées au moins en partie dans le régime non fluidisé (ou compact), autrement dit un régime dans lequel les particules sont essentiellement immobiles les unes par rapport aux autres. Par exemple la fraction volumique occupée par les particules, dans le lit de particules non fluidisé ou compact, peut être de 0,4 à 0,6 (tout particulièrement pour des particules sphériques ou quasi-sphériques).

Le passage entre le régime fluidisé et le régime non fluidisé peut notamment être effectué par un changement de débit du fluide dans la zone de capture.

Ainsi, le débit de fluide est avantageusement contrôlé pendant au moins une étape, et de préférence pendant plusieurs étapes du procédé ou toutes les étapes du procédé.

Généralement, les valeurs de débit avec lesquelles les particules sont dans un état compact sont inférieures de 5 à 50 fois, voire jusqu'à 500 fois par rapport aux valeurs de débit avec lesquelles le lit de particules est dans un état fluidisé, dense mais non compact.

Le débit de fluide dans la zone de capture peut prendre des valeurs différentes au cours du procédé. De préférence, ces valeurs suivent un enchaînement préétabli, et corrélé à l'exécution d'au moins certaines des étapes (a) à (c).

Alternativement, le passage entre les régimes fluidisés et non fluidisés peut être effectué en modifiant un autre paramètre opérationnel que le débit de fluide, et notamment la force s'opposant à l'écoulement. Ainsi, ce paramètre opérationnel peut être un champ électrique, un courant, un champ magnétique, une intensité de vibration mécanique, une pression...

L'application d'une vibration mécanique peut également contribuer à contrôler le caractère fluidisé ou non-fluidisé des particules dans la zone de capture, indépendamment ou en combinaison avec la variation du débit de l'écoulement ou de la force s'opposant à l'écoulement.

### Particules

Les tailles de particules indiquées dans la demande sont des tailles moyennes (Dv50).

Généralement, la taille des particules est adaptée en fonction des dimensions du système fluidique utilisé, et des débits de fluide envisagés.

Les particules peuvent notamment être millimétriques, c'est-à-dire avoir une taille allant de 1 mm à 10 mm ; ou micrométriques, c'est-à-dire avoir une taille allant de 1 µm à 1 mm ; ou nanométriques, c'est-à-dire avoir une taille allant de 1 nm à 1 µm.

De préférence, les particules ont une taille de 50 nm à 50 µm, plus préférentiellement de 100 nm à 1 µm ; ou, alternativement, de 500 nm à 100 µm, plus préférentiellement de 1 µm à 50 µm, ou encore préférentiellement de 100 nm à 10µm.

Il est possible d'utiliser des particules présentant une distribution monomodale ou multimodale.

Par exemple, on peut utiliser des particules ayant une distribution bimodale. Selon certains modes, cette distribution comporte à la fois des particules micrométriques et des particules nanométriques, ou plusieurs types distincts de particules micrométriques, millimétriques ou nanométriques. Préférentiellement, les particules mises en œuvre dans la divulgation sont porteuses de ligands permettant de se lier de façon spécifique à certains organismes.

Selon un mode de réalisation, cette liaison spécifique peut se faire par l'intermédiaire de sites de reconnaissance présents à la surface desdits organismes, ou associés aux dits organismes.

Selon un autre mode de réalisation, la liaison spécifique peut s'effectuer de façon indirecte. Dans ce cas, un ligand spécifique d'un type d'organisme, porteur d'un site de reconnaissance secondaire, est mis en présence d'une part de l'échantillon contenant les organismes, et d'autre part des particules.

Par « *ligand* »*,* on entend une espèce, un ensemble ou sous-ensemble moléculaire, une fonction, un élément de surface ou de volume, présentant une affinité pour un site de reconnaissance. L'affinité mise en jeu peut notamment être de nature chimique, physique ou biologique. Elle peut en particulier reposer sur une interaction électrostatique, magnétique, biochimique, hydrophile-hydrophobe, sur une empreinte moléculaire, une hybridation, une formation de structure supramoléculaire, une agrégation, une association par déplétion, etc.

Selon un mode de réalisation préféré, les ligands sont des anticorps, dirigés contre un antigène de surface des organismes dont il est question.

Selon un autre mode de réalisation préféré, les ligands présentent une affinité pour des éléments glycosylés présents sur la surface des organismes, ou encore des éléments de la matrice extracellulaire.

Egalement, selon certains modes privilégiés, on peut mettre en œuvre plus d'un type de ligands, par exemple si on souhaite capturer en une seule fois plusieurs types d'organismes.

Les particules peuvent être des particules magnétiques, de préférence super-paramagnétiques, en particulier lorsque la force s'opposant à l'écoulement pour la génération du lit fluidisé est magnétique.

Les particules peuvent également être chargées ou diélectriques, en particulier lorsque la force s'opposant à l'écoulement pour la génération du lit fluidisé est électrique.

Les particules peuvent présenter une différence de densité par rapport au fluide présent dans la zone de capture. Par exemple, les particules peuvent présenter une densité supérieure ou égale à celle du fluide, de préférence supérieure ou égale à 1,2 fois la densité du fluide, ou supérieure ou égale à 1,5 fois la densité du fluide, ou supérieure ou égale à 2 fois la densité du fluide, ou supérieure ou égale à 3 fois la densité du fluide, ou supérieure ou égale à 4 fois la densité du fluide.

Alternativement, le fluide peut présenter une densité supérieure ou égale à celle des particules, de préférence supérieure ou égale à 1,2 fois la densité des particules, ou supérieure ou égale à 1,5 fois la densité des particules, ou supérieure ou égale à 2 fois la densité des particules, ou supérieure ou égale à 3 fois la densité des particules, ou supérieure ou égale à 4 fois la densité des particules.

Le procédé de l'invention est avantageusement mis en œuvre avec une quantité de particules plus faible que dans l'art antérieur, grâce au gain de sensibilité permis par l'invention.

Ainsi, selon certains modes de réalisation, la masse totale de particules retenue dans la zone de capture est inférieure à 1 g, de préférence inférieure à 100 mg, de préférence encore inférieure à 10 mg, de préférence encore inférieure à 1 mg, de préférence encore inférieure à 100 µg, et dans certains cas inférieure à 10, 5, 4, 3, 2 voire 1 µg.

### Système fluidique de l'invention

Avantageusement, l'ensemble du procédé de l'invention est mis en œuvre dans un même système fluidique, tel que décrit dans la revendication 9. Cela permet d'éviter de transférer un fluide d'un contenant à un autre via un passage à l'air libre, ou par pipetage, ce qui augmente les risques de contamination, et rend l'automatisation du procédé plus complexe.

Le système fluidique comporte au moins une chambre pour la zone de capture, qui est équipée d'au moins une entrée et une sortie de fluide.

Le système fluidique peut comporter une chambre unique ou plusieurs chambres. Lorsque plusieurs chambres sont utilisées, elles sont alors en connexion fluidique, c'est-à-dire qu'elles sont susceptibles d'échanger du liquide de manière continue grâce à au moins un conduit intermédiaire.

Selon un mode de réalisation, le système fluidique comporte une deuxième chambre en connexion fluidique avec la chambre comportant la zone de capture.

Selon un mode de réalisation, le système fluidique comporte des moyens d'amplification moléculaire et de détection des produits de ladite amplification moléculaire, qui en particulier peuvent être associés à la deuxième chambre. L'amplification moléculaire est décrite plus en détail ci-dessous.

La partie du système fluidique dans laquelle circulent les fluides est de préférence monolithique, c'est-à-dire constituée d'un seul matériau. D'autres matériaux peuvent être utilisés pour les autres éléments du système fluidique, tels que des électrodes, des aimants, des connecteurs, des adhésifs, des éléments de fixation, etc.

De préférence, la chambre comportant la zone de capture ne comporte pas de filtre en entrée et en sortie susceptibles de retenir les particules. Dans l'art antérieur, des filtres sont utilisés pour retenir des particules, ce qui présente des risques de colmatage en présence de fluides contenant des contaminants tels que des débris ou autres pouvant avoir une taille supérieure à celle des organismes à détecter ou des particules.

Le système fluidique peut comporter une pluralité de chambres identiques ou différentes, afin de fournir un fonctionnement en parallèle, en série, ou mixte (avec des circuits parallèles comportant des chambres en série, ou des circuits en série comportant des chambres en parallèle).

Les différentes chambres peuvent être alimentées avec des échantillons, des particules et des milieux identiques ou différents.

Le système comporte en outre avantageusement un ou plusieurs réservoirs, et en particulier au moins un réservoir contenant le milieu de croissance, un réservoir pour l'échantillon, un réservoir pour les particules, optionnellement un réservoir pour une solution de lavage, et éventuellement des réservoirs pour des fluides supplémentaires.

La chambre du système fluidique comportant la zone de capture présente généralement une entrée de fluide, une sortie de fluide et une zone de canal élargie par rapport à l'entrée et à la sortie. Les éventuelles autres chambres du système fluidique peuvent également avoir une géométrie du même type.

Les chambres peuvent également avoir d'autres formes, comme par exemple des formes de cylindre, de parallélépipède, ou des formes plus complexes. Avantageusement, les chambres peuvent comporter des éléments d'homogénéisation d'écoulement. Ces éléments d'homogénéisation peuvent être constitués par des structures multiples attachées aux parois de la chambre, telles que des piliers, de sorte à créer des obstacles locaux à l'écoulement et à empêcher le développement d'un écoulement de type Poiseuille dans la chambre.

Le système fluidique ou des parties de celui-ci, peuvent être fabriqués par impression 3D. Alternativement, le système fluidique de l'invention ou des parties de celui-ci, peuvent être fabriqués par toutes techniques de mise en forme de matériau, telles que l'usinage, le micro-usinage, le moulage sous pression ou par injection, la microlithographie, l'ablation laser, la gravure sèche ou humide.

Le système fluidique et notamment les chambres décrites ci-dessus, peuvent être constitués de toutes sortes de matériaux, tels que les matières plastiques, le verre, les métaux. Selon certains modes préférés, le système fluidique comporte au moins une fenêtre permettant l'imagerie ou la détection optique.

Le système fluidique peut être constitué de plusieurs éléments assemblés les uns aux autres. Selon un mode de réalisation, l'élément comprenant la chambre qui comporte la zone de capture est amovible par rapport au reste du système fluidique. Cela peut permettre d'utiliser une chambre avec zone de capture à usage unique. Le système fluidique comprend dans ce cas des moyens pour effectuer une connexion d'au moins une entrée fluidique et une sortie fluidique de l'élément amovible avec des orifices de circulation de fluide intégrés au reste du système fluidique.

Le système fluidique utilisé dans l'invention comporte avantageusement des moyens de régulation de la température, en particulier dans la zone de capture. Ces moyens peuvent par exemple consister en un chauffage, ou un refroidissement, ou des moyens combinés de chauffage et de refroidissement. A titre d'exemple, il peut s'agir de modules Pelletier, ou d'une circulation de fluides dans un ou des circuits annexes en contact thermique avec le dit système. Il peut aussi s'agir de moyens de chauffage par couche résistante, comme par exemple une couche d'oxyde d'indium et d'étain (ITO). Avantageusement, ces moyens sont régulés par un régulateur de température. Avantageusement, également, ce régulateur de température utilise les informations fournies par un ou plusieurs capteurs de température intégrés au système.

Ainsi, la température peut être régulée, notamment lors de l'étape (b) du procédé, afin de favoriser la croissance des organismes présents dans la zone de capture. Une température de 20 à 60°C, de préférence de 30 à 50°C, plus particulièrement de 35 à 40°C, et par exemple d'environ 37°C, est préférée pour certains organismes.

De préférence, le système fluidique est transportable. Il peut par exemple peser moins de 50 kg, ou moins de 20 kg, ou moins de 10 kg.

De préférence, le système fluidique est portable. Il peut par exemple peser moins de 5 kg, ou moins de 1 kg.

Selon d'autres modes de réalisation, le système utilisé dans l'invention ne présente aucune dimension supérieure à 50 cm, et de préférence aucune dimension supérieure à 30 cm.

La zone de capture du système fluidique, ainsi que les moyens permettant de retenir les particules dans la zone de capture, ont été décrits ci-dessus.

Le système fluidique comporte des moyens de transport des fluides (et des particules), qui sont décrits plus en détail ci-dessous.

Le système fluidique comporte également des moyens de détection, qui sont décrits plus en détail ci-dessous.

### Système microfluidique

De manière particulièrement préférée, le système fluidique est un système microfluidique, c'est-à-dire un système comprenant une ou plusieurs microstructures sur la surface d'un substrat, qui constituent des éléments adaptés pour contenir et / ou diriger des fluides. Ces microstructures ont au moins une dimension qui est inférieure à 1 mm, et de manière plus particulièrement préférée inférieure à 500 µm. Dans certains cas, ces microstructures peuvent avoir au moins une dimension inférieure à 200 µm, ou à 100 µm, ou à 50 µm, ou à 20 µm, ou à 10 µm, ou à 5 µm, ou à 2 µm ou à 1 µm.

Ces microstructures peuvent comporter des volumes fermés ou dans certains cas présenter une surface ouverte.

On désigne par canaux (ou micro-canaux) des microstructures adaptées pour la circulation / l'écoulement de fluides. Ils sont le plus souvent fermés sur l'ensemble du parcours des fluides. Le substrat est de préférence une plaque ou plaquette. Le substrat est de préférence essentiellement rigide, ce qui signifie qu'il peut être manipulé et fixé pour être maintenu immobile, vis-à-vis d'un détecteur par exemple. Il peut être fait de verre, silicium, céramique, métal ou matériau polymérique / plastique. Le substrat peut être recouvert d'un couvercle de même nature, ou d'un matériau flexible, tel qu'un élastomère silicone, par exemple du polydiméthylsiloxane. Alternativement, l'ensemble du substrat et du couvercle peuvent être en un matériau flexible, tel qu'un élastomère silicone, par exemple du polydiméthylsiloxane. On peut également utiliser un polymère fluoré tel que ceux connus sous le nom « Dyneon ». On peut aussi employer un polymère thermoplastique tel qu'un polymère ou copolymère oléfine, notamment oléfine cyclique, polycarbonate, polyméthylméthacrylate, polystyrène, polyéthylène téréphtalate. Les polymères transparents sont préférés, en combinaison éventuellement avec le verre.

La fabrication des microstructures du système microfluidique peut être basée sur des techniques de microfabrication, telles que les techniques de dépôt de film, de photolithographie, de gravure (chimique ou plasma), de thermoformage, de moulage, de moulage par injection, et de collage. Le dépôt de film peut être effectué par centrifugation, par oxydation thermique, par dépôt chimique ou physique en phase vapeur (CVD et PVD), par CVD à basse pression, par CVD améliorée par plasma, par pulvérisation...

Le système microfluidique peut être ou comprendre un laboratoire sur puce.

Le système microfluidique peut comporter un réseau de canaux, c'est-à-dire une pluralité de canaux disposés entre le substrat et son couvercle, ou intégralement entourés par le substrat, et qui sont en communication fluidique soit entre eux, soit avec une ou plusieurs sources de fluide extérieures au système.

Le système microfluidique peut également comporter une série de canaux, c'est-à-dire un ensemble d'une pluralité de canaux non connectés, ou de réseaux de canaux non connectés, sur le même substrat.

Le système microfluidique peut être relié à des réservoirs de fluides ou d'échantillons et autres dispositifs annexes par des tubes ou tuyaux ou connecteurs (par exemple en Y ou en X), afin d'amener des fluides vers, ou de collecter les fluides issus du système. Alternativement, ces tubes ou tuyaux et éventuellement les réservoirs et autres dispositifs annexes peuvent être considérés comme faisant partie du système microfluidique.

Il peut être avantageux d'utiliser un système microfluidique avec des débits de fluides relativement élevés au regard des dimensions en cause, afin de combiner une haute sensibilité avec de faibles dimensions.

Le débit de l'échantillon peut valoir en particulier de 1 nL/min à 10 mL/min, des gammes de débit préférées étant de 1 µL/min à 100 µL/min, de 100 µL/min à 1 mL/min et de 1 à 10 mL/min.

La zone de capture peut avoir un volume allant de 1 nL à 10 mL. Des gammes possibles de volume sont : de 1 mL à 10 mL, ou de 100 µL à 1 mL, ou de 10 µL à 100 µL, ou de 1 µL à 10 µL, ou de 100 nL à 1 µL, ou de 10 nL à 100 nL, voire de 1 nL à 10 nL.

Le système fluidique peut être un système millifluidique, c'est-à-dire qu'il comporte un ou des canaux pour l'écoulement de fluides, dont une dimension au moins est inférieure à 1 cm, de préférence compris entre 1 mm et 5 mm (aucune dimension n'étant toutefois inférieure à 1 mm, comme c'est le cas dans un système microfluidique).

### Transport des fluides et des particules

Dans le procédé de l'invention, les particules doivent être amenées jusqu'à la zone de capture, puis divers fluides doivent circuler à travers la zone de capture, notamment l'échantillon étudié, et le milieu de croissance. D'autres fluides peuvent circuler à travers la zone de capture pour des étapes intermédiaires de lavage, ou pour mener à bien l'étape (c) de détection. Les particules doivent généralement être retirées de la zone de capture, soit pour les besoins de l'étape (c) de détection, soit après celle-ci.

Selon la divulgation, le transport des fluides (et des particules) peut être effectué au moyen d'un système d'actionnement de fluide, qui peut notamment comporter des pompes, des contrôleurs de pression, des éléments vibreurs, des vannes. Dans certaines variantes, le transport des fluides et/ou des particules peut également être effectué au moins partiellement par l'effet de la pesanteur, ou d'une force centrifuge.

A titre de pompes, on peut utiliser notamment des pompes microfabriquées ou des pompes externes, telles que des pompes de contrôle microfluidique, des pompes de seringue, des pompes péristaltiques, des pompes à membrane, des pompes à piston, des pompes rotatives.

De préférence, on utilise des pompes sans impulsion, telles que des pompes microfluidiques contrôlées par pression. De tels systèmes évitent les problèmes d'hystérésis qui peuvent se rencontrer avec les systèmes de pompage fondés sur un contrôle du volume, tels que les pompes à seringue. En effet dans ces derniers, chaque arrêt du flux a tendance à engendrer un empilement compact de particules magnétiques vers l'entrée du canal, bouchant l'entrée du canal et pouvant conduire à une accumulation de pression, puis à un éclatement de l'empilement compact de particules et à la perte de particules lorsque le débit est rétabli.

Les pompes microfluidiques contrôlées par pression, comme par exemple le système MFCS de Fluigent, ou la pompe Mythos de Dolomite, permettent d'éviter d'accumuler une pression excessive, et de limiter les risques ci-dessus.

Le système comporte de préférence un moyen de mesure de pression, ou un moyen de mesure de débit dans la zone de capture, et une combinaison de moyens de mesure de pression et de débit.

Il est préféré de recourir à des systèmes dans lesquels la pression est régulée de manière dynamique grâce aux informations provenant d'un débitmètre. Cela peut permettre d'augmenter progressivement la pression afin d'augmenter ou diminuer rapidement le débit de fluide à travers le lit de particules afin de maintenir le débit dans des limites prédéterminées et ainsi d'éviter tout phénomène d'éclatement du lit de particules.

En effet, avec ce mode de réalisation, on peut arrêter le flux, ou réduire le débit à une faible valeur, tout en conservant le champ de force opposé à l'écoulement, et appliquer une pression positive finie et contrôlée à l'entrée de la chambre. Ainsi, on évite le retour en arrière des particules en amont de la zone de capture.

Le transport des fluides et/ou des particules peut également être effectué par application d'un champ électrique, en générant des forces électrophorétiques, diélectrophorétiques ou électroosmotiques. En particulier, l'écoulement des fluides peut être électroosmotique. Il peut être généré directement par la présence de charges électriques sur des parois du système fluidique ou sur des particules contenues dans le système fluidique (soit dans la zone de capture, soit hors de la zone de capture).

Lorsque le transport des fluides et/ou des particules est effectué par application d'un champ électrique, celui-ci peut être continu, alternatif, pulsé, ou présenter une composante continue et une composante non continue. Il peut avoir notamment une amplitude de 1 à 10 V/cm, ou de 10 à 100 V/cm, ou encore de 100 à 1000 V/cm ; dans d'autres cas, et notamment en présence d'électrodes isolées des fluides, ou lorsque le champ électrique présente un gradient, l'amplitude peut être de 1.000 V/cm à 10.000 V/cm, ou de 10.000 à 100.000 V/cm.

Selon un mode de réalisation, on applique au système fluidique ou à des canaux d'amenée de fluide vers le système fluidique, une vibration mécanique. Cette vibration peut notamment avoir une fréquence valant de 1 Hz à 10 Hz ; ou de 10 Hz à 100 Hz ; ou de 100 Hz à 1 kHz ; ou de 1 kHz à 10 kHz ; ou de 10 kHz à 100 kHz ; ou de 100 kHz à 1 MHz. La vibration mécanique peut être générée, de façon non limitative, par un vibreur inertiel comportant une masse en mouvement, un élément piézoélectrique, un haut-parleur, un élément électromagnétique, un élément rotatif ou un élément alternatif.

L'application d'une vibration mécanique peut permettre de réduire la présence involontaire de bulles de gaz dans le fluide, et ainsi améliorer la qualité de l'écoulement. Elle peut aussi permettre d'induire une composante vibratoire ou alternative dans l'écoulement, qui peut faciliter le contrôle de la fixation des organismes sur les particules, notamment en accélérant la cinétique de contact ; ou qui peut au contraire détacher des organismes éventuellement fixés aux particules par affinité non spécifique.

Dans le cadre du procédé de l'invention, des périodes de circulation continue de fluide peuvent être alternées avec des périodes d'arrêt de fluide (pouvant notamment permettre des étapes d'incubation ou de réaction). Des périodes de circulation de fluide à un premier débit peuvent aussi être alternées avec des périodes de circulation de fluide à un deuxième débit qui est de préférence au moins 10 fois, ou au moins 50 fois inférieur au premier débit.

Des valeurs de débit pouvant être utilisées sont notamment de 1 nL/min à 10 mL/min, et en particulier de 1 µL/min à 100 µL/min.

### Système microfluidique avec capture magnétique

Dans un mode de réalisation préféré, le système fluidique utilisé dans l'invention est un système microfluidique permettant la capture magnétique de particules dans la zone de capture, grâce à des moyens d'application de champ magnétique.

En faisant référence à la **figure 1**, le système microfluidique 1 utilisé dans l'invention peut ainsi comporter au moins une chambre 2 pour l'écoulement d'un fluide avec une entrée 4 et une sortie 5, ainsi que des moyens d'application de champ magnétique 6.

Il peut se présenter sous la forme d'un ensemble comprenant d'une part le dispositif de circulation des fluides (substrat microstructuré comportant la chambre, et les tubulures, réservoirs et autres éléments en connexion fluidique avec celui-ci) et d'autre part les moyens d'application de champ magnétique, non nécessairement fixés ou liés au dispositif de circulation des fluides.

Les termes d'entrée et de sortie sont choisis en référence au sens majoritaire de l'écoulement des fluides (échantillon, milieu de croissance...). Pour les besoins de certains protocoles, on peut toutefois être amené, de façon transitoire, à faire circuler certains fluides dans le sens opposé (de la sortie vers l'entrée), ou entre des entrées et sorties différentes, par exemple pour des opérations de rinçage.

La chambre peut comporter une pluralité d'entrées et / ou une pluralité de sorties, dans l'hypothèse où il s'agit d'un canal ramifié.

En faisant à nouveau référence à la **figure 1****,** la chambre 2 comporte la zone de capture 3, qui est adaptée pour contenir un lit de particules magnétiques (notamment sous forme de lit fluidisé).

La chambre 2 présente une forme généralement élongée, avec un axe longitudinal 7 entre l'entrée 4 et la sortie 5.

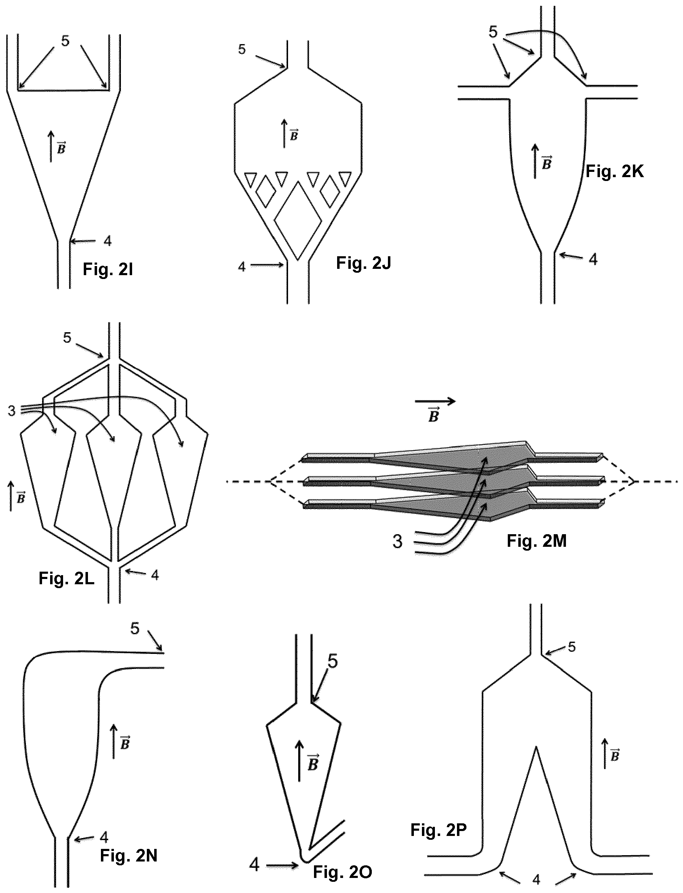

L'axe longitudinal 7 est généralement une droite (au moins dans la zone de capture 3 de la chambre 2), comme illustré par exemple sur la **figure 1****,** mais il peut dans certains cas être composé de segments de droites ou être courbe dans l'hypothèse où la chambre comporte des coudes ou virages ou changements de direction (voir par exemple la **figure 2N****).**

L'axe longitudinal 7 de la chambre 2 correspond généralement à la direction moyenne d'écoulement du fluide dans la chambre (pouvant être définie comme la direction du vecteur vitesse moyen du fluide dans la chambre, en mode d'écoulement non-turbulent).

L'axe longitudinal 7 peut être un axe de symétrie de la chambre 2, ou au moins de la partie de la chambre 2 formant la zone de capture 3.

On définit la section transversale de la chambre comme étant la section orthogonale à l'axe longitudinal 7 de la chambre 2.

Il est préféré que la section transversale de la chambre 2 soit supérieure ou égale (de préférence supérieure) en tout point à la section transversale de l'entrée 4 de la chambre ; et / ou que la section transversale de la chambre 2 soit supérieure ou égale (de préférence supérieure) en tout point à la section transversale à la sortie 5 de la chambre 2.

La zone de capture 3 s'élargit le long de l'axe longitudinal 7, dans le sens de l'écoulement, c'est-à-dire que la section transversale de la chambre 2 croît le long de l'axe longitudinal 7 de l'entrée 4 vers la sortie 5 de la chambre 2.

Par exemple, la chambre peut avoir une forme conique selon son axe longitudinal. Alternativement, et de manière préférée pour une plus grande simplicité de fabrication, la chambre peut avoir une section transversale rectangulaire, présentant une hauteur (ou épaisseur, dans la direction perpendiculaire au plan du substrat) et une largeur, la largeur étant croissante dans le sens de l'écoulement (la hauteur restant constante), ou la hauteur étant croissante dans le sens de l'écoulement (la largeur restant constante), ou la largeur et la hauteur étant croissantes dans le sens de l'écoulement.

La chambre a avantageusement une hauteur (épaisseur) constante pour plus de simplicité de fabrication.

De manière générale, la chambre peut avoir une section transversale circulaire, ovale, triangulaire, carrée, rectangulaire ou autre (y compris différentes formes à différentes positions le long de l'axe longitudinal), et elle peut être ouverte sur un côté vers l'environnement externe (le côté supérieur), et ce sur toute la longueur de la chambre ou seulement une partie de celle-ci. La chambre peut également, de préférence, être fermée, à l'exception de l'entrée et de la sortie.

La chambre peut être un canal capillaire.

La croissance de la section transversale est de préférence continue, et par exemple linéaire.

En aval de la zone de capture, la chambre peut comporter une zone aval, qui est donc située entre la zone de capture et la sortie. Cette zone aval peut avoir une section transversale constante ou croissante, mais également, de préférence, une section transversale décroissante vers la sortie, afin de fournir la transition nécessaire vers la sortie qui présente généralement une dimension réduite.

Préférentiellement la zone de capture a une forme généralement allongée. Préférentiellement, l'ensemble de la chambre a une forme généralement allongée.

Préférentiellement, la longueur de la chambre, et / ou la longueur de la zone de capture, est supérieure à la dimension maximale de la chambre dans sa section transversale, et notamment d'un facteur d'au moins 2, ou d'au moins 3 ou d'au moins 5, et pouvant aller jusqu'à 20, 100 ou 500.

La zone de capture peut être dans certains cas ramifiée, auquel cas la section transversale est constituée par la somme des sections transversales des différentes ramifications.

La dimension maximale de la section transversale peut être par exemple, selon les modes de réalisation, inférieure ou égale à 5 mm, ou à 1 mm, ou à 500 µm, ou à 200 µm, ou à 100 µm, ou à 60 µm, ou à 50 µm, ou à 40 µm, ou à 30 µm, ou à 20 µm, ou à 10 µm, ou à 3 µm, ou à 1 µm, ou à 300 nm, ou à 100 nm, ou à 30 nm, ou à 10 nm.

En outre, le rapport de la longueur de la zone de capture (dimension selon l'axe longitudinal) sur la dimension maximale de la section transversale peut par exemple valoir de 1 à 500, et préférentiellement de 2 à 50, plus particulièrement de 3 à 5, de 5 à 20 ou plus rarement de 20 à 50.

La hauteur ou épaisseur de la chambre peut aller en général de 1 µm à 5 mm, de préférence de 10 µm à 100 µm ou de 100 µm à 1 mm.

La zone de capture de la chambre peut avoir un volume allant jusqu'à 10 mL. Il est toutefois préféré qu'elle présente un faible volume, par exemple de 1 mL à 10 mL, ou de 100 µL à 1 mL, ou de 10 µL ou 100 µL, ou de 1 µL à 10 µL, ou de 100 nL à 1 µL, ou de 10 nL à 100 nL, ou même de 1 nL à 10 nL. Les volumes inférieurs à 10 µL sont préférés.

Il peut être approprié que la chambre, et notamment sa zone de capture, ou une zone de la chambre située en aval de la zone de capture, soit fermée sur l'un de ses côtés par un matériau transparent présentant une épaisseur compatible avec une observation optique, et notamment une observation microscopique à haute résolution, formant une fenêtre. L'épaisseur de la fenêtre est de préférence inférieure à 500 µm, notamment inférieure à 200 µm, en particulier pour une observation microscopique.

Dans certains autres modes de réalisation, préférés par exemple pour des applications à bas coût, une observation microscopique est inutile, et la chambre dispose en ce cas de parois d'épaisseur supérieure à 200 µm, voire supérieure à 500 µm.

Les **figures 2A à 2P** illustrent diverses variantes pour la forme de la chambre décrite ci-dessus, l'entrée (ou les entrées) étant référencée(s) 4 et la sortie (ou les sorties) étant référencée(s) 5. La direction générale du champ magnétique est illustrée par un vecteur sur les schémas.

La **figure 2A** montre une chambre avec une seule entrée 4 et une seule sortie 5, une épaisseur constante, une longueur L, et une largeur I qui augmente linéairement depuis un minimum en entrée 4, jusqu'à un maximum (inférieur à la longueur L), puis diminue linéairement jusqu'à la sortie 5. La forme globale de la chambre est donc celle d'un losange asymétrique. La zone de capture 3 est située entre l'entrée 4 et la zone de la chambre de largeur maximale.

La **figure 2B** montre une variante avec une largeur qui varie de manière non-linéaire. En outre, une fenêtre 15 (telle que décrite ci-dessus) est ménagée sur une surface supérieure ou inférieure de la chambre, et / ou une fenêtre 16 est ménagée en aval de la chambre.

La **figure 2C** montre une variante dans laquelle la chambre présente un élargissement tridimensionnel, en forme de cône.

La **figure 2D** montre une variante dans laquelle la chambre présente un élargissement tridimensionnel, de forme pyramidale.

La **figure 2E** montre une variante de chambre proche de celle de la **figure 2B****.**

La **figure 2F** montre une variante de chambre proche de celle de la **figure 2A****,** mais avec une forme de losange essentiellement symétrique.

La **figure 2G** montre une variante avec une entrée 4 ramifiée en trois canaux d'alimentation, en amont de la chambre.

La **figure 2H** montre une variante avec quatre sorties 5, débouchant sur des canaux avals respectifs parallèles. Dans cette variante, la largeur de la chambre croît continûment de l'entrée 4 jusqu'aux sorties 5, sans passer par un maximum, et la forme de la chambre vue de dessus est donc globalement triangulaire.

La **figure 2I** montre une variante proche de celle de la **figure 2H****,** avec seulement deux sorties 5.

La **figure 2J** montre une variante avec de multiples ramifications en aval de l'entrée 4, de type delta.

La **figure 2K** montre une variante avec trois sorties 5, débouchant sur des canaux avals dans des directions divergentes.

La **figure 2L** montre une variante dans laquelle la chambre est ramifiée en trois branches entre l'entrée 4 et la sortie 5, disposées parallèlement dans le même plan du substrat du système microfluidique, chaque branche comportant une zone de capture 3 (avec élargissement de la branche de l'entrée 4 vers la sortie 5).

La **figure 2M** montre une variante dans laquelle la chambre est ramifiée en trois branches entre l'entrée 4 et la sortie 5, superposées en différentes épaisseurs du substrat du système microfluidique, chaque branche comportant une zone de capture 3 (avec élargissement de la branche de l'entrée 4 vers la sortie 5). Cette disposition permet notamment d'augmenter le débit global du système à partir d'une forme unique, en conservant ses caractéristiques.

La **figure 2N** montre une variante avec une chambre formant un coude au niveau de la sortie 5.

La **figure 2O** montre une variante avec une chambre formant un coude au niveau de l'entrée 4.

La **figure 2P** montre une variante avec une chambre comportant deux entrées 4, la chambre comportant deux branches qui se rejoignent, et une sortie 5 unique en aval du point de jonction.

La **figure 2Q** montre (en coupe horizontale) une variante avec une chambre comportant des éléments d'homogénéisation d'écoulement 17.

En faisant à nouveau référence à la **figure 1****,** l'invention prévoit des moyens d'application d'un champ magnétique 6, le champ magnétique appliqué étant essentiellement parallèle à l'axe longitudinal 7 de la chambre 2. Sur la figure, l'orientation du champ magnétique est illustrée par une flèche.

En particulier, la direction du champ magnétique en tout point de la zone de capture 3 de la chambre 2 forme un angle inférieur ou égal à 20°, ou à 15°, ou à 10°, ou à 5°, par rapport à l'axe longitudinal 7 de la chambre 2 ; ou encore la direction du champ magnétique en tout point de la zone de capture 3 de la chambre est parallèle à l'axe longitudinal 7 de la chambre.

Alternativement, on peut prendre en considération le vecteur moyen du champ magnétique sur une section transversale de la chambre. Ce vecteur moyen forme un angle inférieur ou égal à 20°, ou à 15°, ou à 10°, ou à 5° avec l'axe longitudinal de la chambre, le long de la zone de capture de la chambre; ou encore ce vecteur moyen est parallèle à l'axe longitudinal de la chambre le long de la zone de capture.

Cet alignement du champ magnétique sur la géométrie de la chambre se traduit aussi en termes d'alignement du champ magnétique avec l'écoulement du fluide.

Ainsi, selon des modes de réalisation préférés :
- la direction du champ magnétique en tout point de la zone de capture de la chambre forme un angle inférieur ou égal à 20°, ou à 15°, ou à 10°, ou à 5°, par rapport au vecteur vitesse du fluide dans la chambre en ce point (en écoulement non-turbulent) ;
- le vecteur moyen du champ magnétique sur une section transversale de la chambre forme un angle inférieur ou égal à 20°, ou à 15°, ou à 10°, ou à 5° par rapport à, ou encore est parallèle avec, le vecteur vitesse moyen du fluide sur ladite section transversale, le long de la zone de capture de la chambre.

Une autre manière essentiellement équivalente de définir le parallélisme du champ magnétique par rapport à la zone de capture de la chambre (à quelques divergences ou faibles variations locales ou temporelles près) consiste à imposer que les lignes de champ magnétique dans la zone de capture de la chambre soient essentiellement alignées avec les lignes de champ hydrodynamique dans cette zone de capture (en écoulement non-turbulent), c'est-à-dire que l'angle entre ces lignes de champ respectives est inférieur ou égal à 20°, ou à 15°, ou à 10°, ou à 5° en tout point de la zone de capture.

La composante du champ magnétique dans le plan du substrat perpendiculairement à l'axe longitudinal 7 de la chambre 2 (composante latérale) est en tout point inférieure à 30 %, ou à 20% (ou à 15 %, ou à 10 %, ou à 5 %) de la composante du champ selon l'axe longitudinal, et le champ est donc orienté quasiment selon l'axe longitudinal 7 dans l'ensemble de la chambre 2. Il est à noter que la présence d'une décroissance du champ le long de l'axe longitudinal implique, par conservation du flux, une divergence du champ, et qu'en certains points, et notamment vers les parois de la chambre, le champ présente par conséquent une composante latérale non nulle.

Ce champ magnétique peut être créé au moyen d'un aimant permanent, d'un électroaimant, ou d'une combinaison de ceux-ci, éventuellement en association avec une pièce polaire formée avec un matériau magnétique doux susceptible de diriger les lignes de champ. De préférence, une telle pièce polaire est dépourvue de toute microstructure susceptible de créer une pluralité de maxima locaux du champ magnétique.

Dans le cas de l'utilisation d'un électroaimant sans noyau, par exemple une bobine électrique, on considère que les pôles de l'électroaimant sont les deux plans correspondant à l'entrée et à la sortie du flux magnétique dans la bobine.

Dans la suite, sauf mention contraire, on désignera les aimants permanents et les électro-aimants sous le terme générique « *aimant* »*.*

Dans certains modes de réalisation, le champ magnétique peut être activable ou réglable. En particulier, il est avantageux de pouvoir modifier l'intensité (l'amplitude) du champ magnétique, sans modifier son orientation (c'est-à-dire sa direction, ou la forme des lignes de champ).

Selon un mode de réalisation, la divulgation prévoit de disposer l'aimant avec son axe polaire nord / sud essentiellement aligné avec l'axe longitudinal de la chambre. Il est préféré de disposer l'aimant du côté de l'entrée de la chambre. Cela diffère de géométries proposées dans l'état de la technique, où les aimants ou électroaimants sont disposés sur les côtés de la chambre ou au-dessus et / ou en-dessous de la chambre, c'est-à-dire en tout état de cause près des parois de la chambre essentiellement parallèles à la direction de l'écoulement.

La face de la partie polaire ou de l'aimant permanent faisant face à la chambre de capture peut, selon les modes de réalisation, être plate, ou présenter diverses formes. Par exemple, elle peut être courbée selon une ou plusieurs directions, ou même dans certain cas comporter une ou plusieurs arêtes. Elle est de préférence configurée de façon à générer un champ qui décroit de façon progressive et continue à l'intérieur de la zone de capture. S'il existe des arêtes, elles doivent donc de préférence présenter un angle obtus, préférablement supérieur à 60°, ou supérieur à 80°. De même, si ladite face de la partie polaire ou de l'aimant est courbe et convexe (bombée), elle présente de préférence un rayon de courbure supérieur à la longueur de la portion de la zone de capture destinée à recevoir et à retenir des particules magnétiques. L'ajustement de la forme de la pièce polaire (ou de l'aimant) peut permettre de moduler la divergence du champ magnétique et donc d'optimiser les performances du système, en fonction de la forme de la chambre et de sa zone de capture, soit par essais et erreurs, soit par simulation numérique.

Ainsi, l'utilisation du champ magnétique prévu dans l'invention varie de préférence de manière continue et monotone (décroissante) le long de l'axe longitudinal ou de la direction d'écoulement, de l'entrée vers la sortie, dans la zone de capture. Tout maximum local d'intensité est ainsi évité, qui pourrait conduire à un piégeage local et donc compact de particules magnétiques.

Des variations non linéaires, voire non continues, de la section transversale de la chambre, de la vitesse moyenne d'écoulement du fluide, et de l'intensité du champ magnétique, sont possibles dans certains cas, selon les débits et temps de résidence du fluide souhaités.

Selon un mode de réalisation alternatif, illustré à la **figure 4**, le champ magnétique est essentiellement orthogonal à l'axe longitudinal 7 de la chambre, au lieu d'être essentiellement parallèle. Par exemple, les moyens d'application d'un champ magnétique 6 peuvent comprendre un premier aimant 6a et un deuxième aimant 6b, situés de part et d'autre de la zone de capture 3 de la chambre et se faisant face, le pôle nord du premier aimant 6a étant dirigé vers la chambre et le pôle sud du deuxième aimant 6b étant dirigé vers la chambre.

La face du pôle nord du premier aimant 6a n'est pas parallèle à la face du pôle sud du deuxième 6b, de sorte que l'intensité du champ magnétique soit décroissante dans la zone de capture 3 selon la direction d'écoulement (les faces des aimants sont plus proches du côté amont que du côté aval dans la zone de capture 3).

Dans l'exemple illustré, la zone de capture 3 est délimitée par des côtés de la chambre non parallèles, et qui s'écartant dans le sens de l'écoulement, et les aimants 6a et 6b sont disposés parallèlement à ces côtés de la chambre.

En réglant correctement l'intensité du champ magnétique et le débit de fluide, on réalise un équilibre entre les forces magnétiques et hydrodynamiques permettant de maintenir les particules magnétiques dans la zone de capture.

Le système microfluidique peut aussi comporter une pluralité d'autres canaux, d'entrées et sorties supplémentaires et de vannes, ainsi que des réservoirs des différents fluides utilisés.

Dans l'exemple illustré à la **figure 1**, le système microfluidique 1 comporte un canal d'alimentation 8 connecté fluidiquement à l'entrée 4 de la chambre 2 décrit ci-dessus. Ce canal d'alimentation 8 comporte une première entrée 9, une deuxième entrée 10 et une troisième entrée 11, chaque de ces entrées pouvant être connectée à un réservoir d'un fluide distinct et / ou à des moyens de contrôle de la pression. En sortie 5 de la chambre 2 décrite ci-dessus est connectée une conduite secondaire 13, elle-même connectée à une sortie 12 qui peut être connectée à un réservoir de fluide et / ou à des moyens de contrôle de la pression.

Dans l'exemple illustré, le canal d'alimentation 8 forme un coude d'un angle supérieur à 90° avec la chambre 2 principale, ce qui peut être avantageux pour amener des particules magnétiques depuis un réservoir jusqu'à la chambre 2.

Le caractère régulier de la courbure, combiné à la faible section du canal dans cette zone dans laquelle le champ magnétique n'est pas dirigé selon l'axe du canal, et qui ne présente donc pas le caractère d'une zone de capture, permet d'éviter que certaines particules magnétiques restent piégées dans cette section du canal, et permet au contraire de contraindre la plus grande partie, et dans les cas les plus favorables la totalité, des particules magnétiques contenues dans le canal, à rejoindre la zone de capture 3 et à y rester. Mais toute autre conformation, notamment dans l'alignement de la chambre 2, est possible. De même, dans l'exemple illustré, la conduite secondaire 13 est alignée selon l'axe longitudinal 7 de la chambre 2, mais toute autre conformation est possible.

Le système microfluidique peut aussi être associé à, ou peut comprendre, tout contrôleur informatique, électronique ou électrique, afin par exemple de contrôler la température et le fonctionnement des différents composants, d'automatiser les opérations et d'enregistrer des données.

Selon un mode de réalisation, le système utilisé dans l'invention comporte une conduite secondaire présentant une résistance hydraulique supérieure à celle de la chambre 2, soit en amont (avant l'entrée de la chambre) soit en aval (après la sortie de la chambre), en série par rapport à la chambre (principale) décrite ci-dessus. Ce mode de réalisation permet un meilleur contrôle du flux dans la chambre.

Dans l'exemple de la **figure 1**, la conduite secondaire 13 présente une résistance hydraulique supérieure à celle de la chambre 2, par un choix judicieux de sa longueur et de sa section transversale (qui peut être par exemple égale, ou inférieure ou égale, à la section transversale de la chambre 2 au niveau de sa sortie 5).

De préférence la résistance hydraulique de la conduite secondaire est au moins 2 fois, ou au moins 5 fois, ou au moins 10 fois, par exemple entre 10 fois et 100 fois celle de la zone de capture de la chambre décrite ci-dessus (en l'absence de particules magnétiques).

La conduite secondaire peut être un canal secondaire formé dans le même substrat que la chambre, ou il peut s'agir d'un tube ou tuyau connecté à la chambre.

### Etapes du procédé

Les étapes (a) à (c) du procédé peuvent être séquentielles (et éventuellement séparées dans le temps) ; autrement dit, l'étape (a) est effectuée dans sa totalité ; puis l'étape (b) est effectuée dans sa totalité ; puis l'étape (c) est effectuée dans sa totalité. Alternativement, les étapes peuvent être au moins partiellement effectuées de manière simultanée.

Par exemple, l'étape (c) de détection peut être effectuée concomitamment avec l'étape (a) et/ou l'étape (b). Cela permet par exemple de mesurer l'évolution du nombre ou de la taille des organismes au cours du procédé.

Dans un tel mode de réalisation, le résultat de la détection peut être utilisé pour modifier l'étape (a) et / ou l'étape (b), et notamment pour modifier la durée de l'étape, ou le débit de liquide au cours de l'étape, ou la valeur d'un autre paramètre opérationnel tel que défini ci-dessus.

Selon une variante, le procédé de détection est mis en œuvre avec plusieurs échantillons, auquel cas plusieurs échantillons sont injectés dans la zone de capture au cours de l'étape (a).

### Croissance des organismes

Lors de l'étape (b), on fait circuler dans la zone de capture un milieu de croissance. Ce milieu permet de contrôler la croissance des organismes de l'échantillon éventuellement capturés dans la zone de capture lors de l'étape (a) par fixation aux particules.

De préférence, il s'agit d'un milieu nutritif ou milieu de culture.

Le milieu de croissance peut être spécifique d'un type d'organismes particuliers. Alternativement, il peut s'agir d'un milieu non sélectif.

Par « *croissance* », on entend une amplification métabolique des organismes, c'est-à-dire un processus vital par lequel le nombre ou la masse ou le nombre de cellules (dans le cas d'organismes pluricellulaires) des organismes augmente. Cette croissance peut correspondre à un développement, une culture, une division, une prolifération ou une expansion. De préférence, lorsque les organismes sont des cellules individuelles, la croissance correspond à une succession de divisions cellulaires.

Avantageusement, au moins certains des organismes générés par division cellulaire se fixent aux particules, lors de l'étape (b).

Dans certains modes de réalisation, avant, après ou au cours de l'étape (b), les organismes éventuellement capturés sont mis en présence d'espèces vis-à-vis desquelles on cherche à étudier la réaction desdits organismes.

Ces espèces peuvent être notamment des agents potentiellement toxiques ou au contraire des stimulateurs de croissance ou de division, des agents de signalisation déclenchant certaines réactions métaboliques spécifiques, ou encore des substrats vis-à-vis desquels on cherche à déterminer le potentiel de métabolisation des dits organismes.

Ces espèces peuvent également être d'autres organismes. Ainsi, l'invention permet de mener à bien une co-culture. En particulier, on peut réaliser une co-culture de cellules eucaryotes, notamment de cellules de mammifères ou de plantes, en présence d'autres organismes, et notamment de bactéries ou de fungi. Une application particulière est celle de l'étude de bactériomes.

De préférence, aucune oxygénation du milieu de croissance n'est prévue. Ainsi, on évite la présence de bulles de gaz, ce qui permet un écoulement de fluide plus régulier et mieux contrôlé.

Lors de l'étape (b), les organismes éventuellement capturés dans la zone de capture peuvent subir une croissance exponentielle. Cela offre une large gamme dynamique de détection et rend l'invention très avantageuse, par exemple vis-à-vis des méthodes de culture sur milieu solide de l'art antérieur.

L'échantillon utilisé à l'étape (a), avant la croissance, peut comprendre des organismes cibles dans une concentration inférieure à 1 million par mL (ou par gramme), de préférence inférieure à 1000 par mL (ou par gramme), de préférence encore inférieure à 100 par mL (ou par gramme), de préférence encore inférieure à 10 par mL (ou par gramme), et même éventuellement inférieure à 1/mL (ou par gramme), ou inférieure à 1 organisme pour 10 mL (ou pour 10 g), ou inférieure à 1 organisme pour 25 mL (ou pour 25 g).

L'échantillon utilisé à l'étape (a), avant la croissance, peut contenir un nombre total d'organismes à détecter inférieur à 100.000, de préférence inférieur à 10.000, de préférence encore inférieur à 1.000, de préférence encore inférieur à 100, de préférence encore inférieur à 50, de préférence encore inférieur à 20, de préférence encore inférieur à 10, et dans certaines application de préférence inférieur à 5, 4, 3, 2 voire il peut contenir un seul organisme. L'invention offre ainsi une sensibilité de détection particulièrement intéressante dans les échantillons très dilués.

De préférence, la quantité d'organismes sur laquelle porte l'étape de détection est supérieure à la quantité d'organismes initialement contenue dans l'échantillon, de préférence supérieure à deux fois cette quantité, de préférence supérieure à 10 fois, de préférence supérieure à 100 fois, de préférence supérieure à 1.000 fois, de préférence supérieure à 10.000 fois, et parfois de préférence supérieure à 100.000 fois voire à 1.000.000 fois cette quantité initiale.

### Détection

La détection effectuée à l'étape (c) a pour objet d'identifier si les organismes recherchés sont présents ou non ; ou bien de les dénombrer (ou de déterminer leur concentration dans l'échantillon étudié) ; ou bien de caractériser leur nature ou leur type.

La détection repose sur la mesure d'une propriété. On peut également utiliser une combinaison de plusieurs mesures de propriétés. Chaque mesure peut être de nature directe ou indirecte.

La mesure de propriété peut être effectuée *in situ,* sur le lit de particules situé dans la zone de capture, ou bien *ex situ,* c'est-à-dire hors de la zone de capture.

La mesure *in situ* présente l'avantage de la simplicité et de la compacité. La mesure *ex situ* peut permettre un champ d'analyse plus important, et notamment autoriser un typage des organismes capturés.

Selon un mode de réalisation de la variante *ex situ,* les particules ou une partie des particules contenues de la zone de capture (auxquelles sont éventuellement fixés les organismes à détecter) sont retirées de la zone de capture (par exemple en augmentant le débit de fluide de sorte à vaincre la force s'opposant à l'écoulement, ou en supprimant la force s'opposant à l'écoulement) et transférées dans une autre zone du dispositif, préalablement à l'étape (c) de détection.

Selon un autre mode de réalisation de la variante *ex situ,* les organismes éventuellement fixés aux particules dans la zone de capture sont libérés en tout ou partie des particules (par exemple en faisant circuler dans la zone de capture un milieu altérant l'interaction entre les organismes et les particules), retirés de la zone de capture et transférés dans une autre zone du dispositif préalablement à l'étape (c) de détection.

Selon un autre mode de réalisation de la variante *ex situ,* des espèces sécrétées par les organismes éventuellement fixés aux particules dans la zone de capture ou des constituants de ces organismes sont retirés de la zone de capture et transférés dans une autre zone du dispositif préalablement à l'étape (c) de détection. Dans ce mode de réalisation, on peut prévoir notamment une étape préalable de lyse cellulaire et plus particulièrement de protéolyse.

Par « *espèces secrétées* », on entend tout type d'espèces moléculaires, multimoléculaires, organiques ou cellulaires, produites par les organismes présents dans le lit, naturellement ou par suite de stimuli particuliers, d'apoptose ou de lyse. Il peut s'agir par exemple de protéines ou de peptides excrétés, de facteurs de signalisation, d'exosomes, de métabolites, d'acides nucléiques, de tous types d'agrégats moléculaires, de produits de lyse, mais aussi d'organismes (par exemple cellules) issus de la division ou de la modification des organismes présents dans le lit.

De préférence, la détection de l'étape (c) est effectuée sur au moins 20 % des organismes présents dans la zone de capture à l'issue de l'étape (b), de manière plus particulièrement préférée au moins 50 %, et de manière encore plus préférée au moins 80 %.

La propriété mesurée peut par exemple être une propriété mécanique, hydrodynamique, dimensionnelle, pouvant correspondre à une mesure de force, de déplacement, de pression, de débit, de masse ou de densité.

Il peut également s'agir d'une propriété optique, telle qu'une densité optique, une turbidité, une intensité de rayonnement optique à une ou plusieurs longueurs d'ondes ou dans une gamme de longueurs d'onde, un coefficient d'absorption de rayonnement à une ou plusieurs longueurs d'ondes ou dans une gamme de longueurs d'onde.

La propriété mesurée est par exemple le volume occupé par les particules (et les organismes liés) dans la zone de capture. Il a été constaté que le volume occupé par les particules, au cours de l'étape de croissance, augmente du fait de cette croissance des organismes. Cet accroissement de volume peut être mesuré soit directement soit grâce à un changement de surface ou de longueur du lit de particules selon une ou plusieurs directions.

En particulier, la mesure du temps nécessaire pour que le volume occupé par les particules atteigne une valeur seuil prédéterminée permet de déduire la concentration en organismes présents dans la zone de capture au début de l'étape (b), et donc de déduire la concentration en organismes dans l'échantillon.

La propriété mesurée peut encore être une propriété hydrodynamique, telle qu'une porosité, une viscosité, une élasticité ou une viscoélasticité.

La propriété mesurée peut encore être une propriété de texture, telle qu'une granulosité, ou toute propriété affectant l'apparence visuelle d'une image du lit de particules (ou de la fraction prélevée des particules), ou une fonction pouvant être dérivée de ladite image.

La propriété mesurée peut encore être une propriété optique ; on peut ainsi procéder à une mesure d'intensité lumineuse, d'absorption, de fluorescence ou de luminescence.

L'étape (c) peut également mettre en œuvre une détection biologique ou biochimique, telle qu'une analyse génétique, protéomique ou la mesure d'une activité métabolique.

En particulier, on peut utiliser une mesure de type ELISA (dosage d'immunoadsorption par enzyme liée), immunoagglutination, mesure de concentration en espèces chimiques ou métabolites, séquençage ou génotypage d'ADN ou d'ARN, PCR, hybridation sur des réseaux d'hybridation ou puces à ADN ou puces à protéines, ou tout autre méthode mettant en jeu une étape d'amplification ou d'hybridation d'ADN.

Lorsque la mesure est effectuée *in situ* dans la zone de capture, les mesures directes de propriété physique, ou de propriété de texture, ou de propriété optique, ou d'une combinaison de celles-ci, sont préférées.

Il peut être avantageux d'effectuer la mesure *in situ* sur un lit de particules non fluidisé (compact), par exemple lorsque la mesure porte sur le volume du lit, mais aussi pour certaines mesures biochimiques telles qu'un dosage d'immunoadsorption par enzyme liée.

Dans le cas de la mesure *ex situ,* celle-ci peut par exemple être effectuée le long d'un canal de sortie connecté à la sortie de la zone de capture. Préférentiellement, ce canal de sortie n'est pas connecté à l'entrée de la zone de capture, ce qui simplifie la gestion des fluides.

La détection peut comporter ou être précédée par une étape d'amplification moléculaire. Par « *amplification moléculaire* », on entend un procédé par lequel on obtient, à partir d'une molécule cible, une multiplicité de molécules à détecter.

L'amplification moléculaire peut notamment être une amplification d'acides nucléiques, et tout particulièrement la PCR (réaction de polymérisation en chaîne).

D'autres exemples d'amplification d'acides nucléiques sont la transcription inverse (RT), l'amplification isotherme d'ADN, la réplication circulaire (RCA pour « *rolling circle amplification* »), la réplication circulaire branchée, l'amplification cercle à cercle (C2CA), l'amplification de l'ADN facilitée par boucle (LAMP pour « *loop-mediated amplification »*), l'amplification NASBA (pour « *nucleic acid sequence-based amplification* »), l'amplification TMA (pour « *transcription-mediated amplification* »), l'amplification SMART (pour « *signal-mediated amplification of RNA technology* »), l'amplification HDA (pour « *helicase-dependent amplification* »), l'amplification RPA (pour « *recombinase polymerase amplification* »), l'amplification CPA (pour « cross-priming amplification »), l'amplification SMART-AMP (pour « *Smart amplification* »), l'amplification RAM (pour *« ramification amplification* »), l'amplification SDA (pour « *strand displacement amplification* »), l'amplification NEAR (pour « *nicking enzyme amplification reaction* »), l'amplification NEMA (pour « *nicking enzyme-mediated amplification* »), l'amplification ICA (pour « *isothermal chain amplification* »), l'amplification EX-PAR (pour « *exponential amplification reaction* »), l'amplification BAD AMP (pour « *beacon-assisted détection amplification* ») et l'amplification utilisant l'enzyme Phi29 DNA polymerase. On pourra se reporter à l'article de Niemz et al. dans Trends in Biotechnology, 29:240-250 (2011) à ce sujet.

L'amplification moléculaire peut également être une amplification enzymatique. Dans ce cas, on utilise une enzyme se liant de façon spécifique (directe ou indirecte) à une molécule cible, et susceptible de transformer une multiplicité de molécules d'un substrat, cette transformation pouvant être détectée plus aisément que la molécule cible initiale. Un exemple d'amplification enzymatique est la technique ELISA.

L'amplification moléculaire peut également être une amplification PLA (pour « *proximity ligation assay* ») ou reposer sur une hybridation multiple et/ou multiplexée. Des kits pour mettre en œuvre de telles techniques sont disponibles dans le commerce sous les marques QuantiGene® ou NanoString®.

L'amplification moléculaire peut également être de type catalytique.

L'amplification moléculaire peut également être une technique de chimiluminescence, d'électrochimiluminescence ou d'électrochimie.

Le procédé de l'invention peut permettre le typage des organismes de l'échantillon.

Ce typage peut notamment être un typage cellulaire.

Le typage cellulaire peut consister à déterminer la souche particulière des organismes (par exemple de bactéries), ou à déterminer une classe, un groupe, une espèce, une variété de ces organismes, ou encore toute autre caractéristique de la classification des espèces.

Le typage cellulaire peut également permettre de détecter une anomalie de certaines cellules parmi d'autres, par exemple pour des cellules d'organismes pluricellulaires.

Le typage peut également consister à déterminer une ou plusieurs propriétés d'intérêt d'organismes génétiquement modifiés.

Le typage peut aussi être un génotypage. Il peut consister à déterminer le génotype d'organismes pathogènes, de cellules cancéreuses, de cellules fœtales. Il peut consister à identifier des mutations ponctuelles ou des modifications de génome à grande échelle, telles que délétions, amplifications, réarrangements ou insertions.

Le typage peut aussi être un phénotypage. Il peut consister à déterminer la morphologie, la structure, le protéome ou le transcriptome des organismes, on encore à déterminer des modifications d'acides nucléiques telles que des méthylations, phosphorylations ou autres modifications d'acides nucléiques intracellulaires.

Le typage peut aussi être un typage fonctionnel. Il peut consister à évaluer la production de certaines protéines, métabolites ou acides nucléiques tels que des micro-ARN, la métabolisation de certaines espèces, ou encore la résistance à certaines espèces, comme en particulier la résistance à des agents toxiques ou des médicaments. Le typage fonctionnel peut également impliquer l'évaluation d'une capacité de prolifération, ou d'une capacité de différenciation, un caractère pluripotent.

Afin de permettre la mise en œuvre de l'étape (c) du procédé, le système fluidique divulgué comporte des moyens adéquats, et en particulier un système de mesure d'au moins une propriété physique, chimique ou biologique.

Ce système de mesure peut être associé à la chambre comportant la zone de capture (pour une mesure *in situ*) ou être associé à une autre chambre du système fluidique (pour une mesure *ex situ*)*,* que l'on peut qualifier de chambre de détection.

Le système de mesure peut comporter un dispositif d'imagerie du contenu du système fluidique et notamment de la chambre de détection ou de la zone de capture. Ce dispositif d'imagerie est de préférence associé à un logiciel d'analyse d'image.

Le système de mesure peut comporter une ou plusieurs sources lumineuses, et un ou plusieurs détecteurs d'intensité lumineuse. Il peut comporter un spectromètre, un analyseur de fréquences, un analyseur d'impédance ou un conductimètre (avec des électrodes).

Le système de mesure peut comporter des moyens de mesure de résistance hydrodynamique, de viscosité, de perméabilité, de pression ou de débit.

Le système de mesure peut encore comporter un dispositif de mesure de propriété biologique, comme un séquenceur, un dispositif d'électrophorèse, un spectromètre de masse, un appareil de PCR, un système à résonance plasmon, une microbalance à quartz ou un fluorimètre.

La mesure effectuée permet de déduire une information relative à la présence, à l'état mort ou vivant, à la concentration ou au type des organismes de l'échantillon.

La déduction de cette information peut être effectuée au moyen d'un programme d'ordinateur, ou éventuellement au moyen d'un abaque ou d'une formule mathématique.

### Applications d'analyse

L'invention permet d'effectuer au moins une opération d'identification, de détection, d'analyse, d'identification, de génotypage, de quantification ou de phénotypage d'organismes présents dans un échantillon.

L'invention peut permettre de déterminer si un échantillon contient des organismes vivants (par opposition à des organismes morts).

L'invention peut également permettre de dénombrer des organismes (ou de déterminer la concentration des organismes dans l'échantillon).

L'invention peut également permettre de déterminer le type d'organismes éventuellement présents dans l'échantillon.

L'invention peut également permettre de déterminer les capacités de développement, de prolifération ou de division des organismes.

L'invention est particulièrement intéressante pour la détection d'organismes choisis parmi les bactéries, les parasites unicellulaires ou pluricellulaires, les champignons, les cellules de mammifères et les cellules de plantes.

Selon des modes de réalisation particuliers, les organismes en question sont des organismes pathogènes ; ou sont des cellules souches ; ou sont des cellules cancéreuses.

L'invention trouve notamment à s'appliquer à la détection de l'effet de molécules et notamment de médicaments sur des cellules ou autres organismes. Pour ce faire, il est possible de prévoir une étape de circulation dans la zone de capture d'un fluide contenant une molécule ou un médicament étudié, après l'étape (a), et notamment : soit entre l'étape (a) et l'étape (b) ; soit au cours de l'étape (b) ; soit après l'étape (b).

L'invention peut être appliquée pour la mise en œuvre d'une analyse diagnostique (en médecine humaine ou vétérinaire).

L'invention peut être appliquée pour la mise en œuvre d'une analyse environnementale, telle que la recherche ou l'identification d'organismes (notamment de bactéries, ou d'organismes pathogènes) dans l'eau ou dans le sol par exemple.

L'invention peut être appliquée pour la mise en œuvre d'une analyse de bactéries présentes chez des animaux et notamment des mammifères (bactériome du tube digestif, bactériome de la peau...).

L'invention peut être appliquée pour l'identification ou la sélection d'organismes génétiquement modifiés. L'invention peut permettre le génotypage ou le phénotypage d'organismes (et notamment d'organismes pathogènes).

L'échantillon utilisé dans le procédé de l'invention peut être un échantillon de fluide corporel tel que le sang, la salive, le liquide céphalorachidien, le liquide amniotique, l'urine, la lymphe, une ponction de biopsie, des selles ou encore un échantillon dérivé de l'un de ces fluides corporels.

L'invention peut notamment être appliquée au diagnostic d'une infection urinaire ; elle peut également être appliquée au diagnostic d'une septicémie.

L'invention peut également être appliquée à la détection d'une infection par un d'agent pathogène, et par exemple à la détection d'une infection par E. *coli*, *Salmonella*, *C. difficile*, *Streptococcus*, ou l'agent du SRAS.

Alternativement, l'échantillon peut être issu d'un aliment, ou encore peut être prélevé dans l'environnement.

A titre d'exemple, le procédé de l'invention peut être appliqué à la détection de la présence des organismes suivants dans les aliments, et notamment dans les produits laitiers (tels que lait en poudre, fromages, beurre, crème glacée...) :
- salmonelles (notamment détection de la présence ou absence d'au moins une salmonelle dans 25 g de produit) ;
- *Enterobacteriaceae* (notamment détection d'une concentration supérieure ou égale à 1 ou à 5 ufc par mL, ou détection de la présence ou absence d'au moins une unité dans 10 g de produit) ;
- *E. coli* (notamment détection d'une concentration supérieure ou égale à 10 ou à 10² ou à 10³ ufc par g) ;
- staphylocoques à coagulase positive (notamment détection d'une concentration supérieure ou égale à 10 ou à 10² ou à 10³ ou à 10⁴ ou à 10⁵ ufc par g) ;
- *Bacillus cereus* (notamment détection d'une concentration supérieure ou égale à 1 ufc par mL, ou à 50 ou à 500 ufc par g) ;
- flore aérobie (notamment détection d'une concentration supérieure ou égale à 1 ufc par mL) ;
- bactéries réductrices des sulfites (notamment détection d'une concentration supérieure ou égale à 1 ufc par mL) ;
- *Listeria monocytogenes* (notamment détection d'une concentration supérieure ou égale à 1 unité dans 25 g de produit).

### Applications préparatives

L'invention peut également être appliquée à la préparation ou à la micro-préparation d'organismes. En effet, elle permet la capture d'un nombre très faible d'organismes, leur multiplication et éventuellement leur caractérisation, le tout dans un dispositif compact et fortement automatisé.

Ainsi, un procédé de préparation d'organismes selon l'invention est mis en œuvre de la même manière que n'importe lequel des procédés de détection décrits ci-dessus, à ceci près que :
- l'échantillon liquide contient des organismes parents susceptibles de se lier aux particules de la zone de capture ;
- on prévoit une étape de collecte, à la sortie de la zone de capture, d'organismes issus de la division des organismes parents.

Dans un tel procédé de préparation, l'étape de détermination de la présence, de la nature ou de la concentration des organismes dans la zone de capture, ou encore l'étape de mesure d'une propriété physique de la zone de capture, sont optionnelles.

Toutefois de manière avantageuse, l'une ou l'autre au moins de ces étapes est effectivement présente, auquel cas le procédé de l'invention est à la fois un procédé de détection d'organismes dans un échantillon liquide et de préparation d'organismes à partir de cet échantillon liquide.

Avantageusement, le procédé de préparation d'organismes selon l'invention est mis en œuvre dans un système fluidique selon l'invention.

Un tel système fluidique comporte ainsi des moyens de collecte, à la sortie de la zone de capture, d'organismes issus de la division des organismes parents. Il peut comporter ou non les moyens de mesure d'une propriété physique de la zone de capture et/ou les moyens de détermination de la présence ou de la concentration ou de la nature des organismes dans l'échantillon décrits ci-dessus en relation avec les systèmes fluidiques dédiés à la détection.

En particulier, la méthode préparative peut être intéressante en biotechnologie, pour sélectionner et préparer des organismes présentant des caractéristiques intéressantes pour une application particulière.

Elle peut également être intéressante pour la sélection et la production de cellules progénitrices, de cellules souches, de cellules hématopoïétiques, de cellules souches hématopoïétiques, ou de cellules endothéliales formatrices de colonies.

Elle peut trouver par exemple des applications pour la capture et la multiplication, à partir d'échantillons de sang de patient, de cellules endothéliales formatrices de colonies, pour des applications de médecine régénérative, ou de capture et multiplication de cellules souches, primaires ou induites.

L'Invention est particulièrement intéressante dans ces applications grâce à sa capacité, démontrée dans l'exemple 5 ci-dessous, de capturer des cellules en fonction de critères spécifiques, puis de libérer en continu des espèces produites par ces cellules, et notamment d'autres cellules, identiques ou différentes, issues des cellules capturées dans un premier temps.

### EXEMPLES

Les exemples suivants illustrent l'invention sans la limiter.

### Exemple 1 - estimation du taux de capture

On réalise un système microfluidique conformément à la **figure 1****.** La chambre 2 fait 21 mm de longueur et 50 µm d'épaisseur, avec une largeur variant de 100 µm à 2 mm (zone de largeur maximale).

Le système microfluidique est préparé en polydiméthylsiloxane selon le protocole de microlithographie décrit dans l'article de Mohamadi et al. dans Biomicrofluidics, 5,044114 (2011).

Pour la génération du champ magnétique, on utilise un aimant permanent de type NdFeB1, placé à proximité de la chambre 2 : la distance entre l'aimant et le système microfluidique est de 2 mm. L'aimant est magnétisé dans sa plus grande dimension et présente un champ magnétique rémanent de 1,47 T. Il présente une taille de 30 x 20 x 20 mm.

Le système est similaire à celui employé dans les exemples 1, 2, 3, 4 et 5 du document WO 2014/037674, auquel il est expressément renvoyé pour plus de détails.

Dans le présent exemple, on s'intéresse à la capture de salmonelles.

Les particules utilisées sont des particules Dynabeads® anti-salmonelles (commercialisées par Life Technologies®), en une quantité totale de 50 µg.

Les organismes étudiés sont des salmonelles (espèce *Salmonella typhimurium*). Les échantillons contenant les salmonelles sont produits par dilution des bactéries dans un tampon phosphate salin avec ajout de 0,1 % d'albumine de sérum bovin (BSA) à partir de colonies cultivées sur boîtes de Pétri.

Pour effectuer la capture des bactéries par le lit de particules on injecte un échantillon à travers la zone de capture avec un débit de 1000 nL/min. Cette étape dure 50 minutes, pour un échantillon de 50 µL (contenant 400 bactéries).

Puis on effectue un lavage avec un tampon phosphate salin avec 0,1 % de BSA. Le débit est réglé à 1500 nL/min, et le volume de liquide injecté est de 40 µL.

Une première estimation du taux de capture des bactéries est réalisée en mesurant la teneur en bactéries dans les liquides récupérés en sortie du système (90 µL correspondant au passage de l'échantillon et du tampon de lavage). La mesure est effectuée par culture sur boîte de Pétri et comptage des colonies formées.

Une deuxième estimation est réalisée en retirant les particules du système microfluidique, et en cultivant les bactéries capturées.

Les deux estimations concordent pour fournir un taux de capture d'environ 90 %.

En outre, la spécificité de la capture des bactéries a déjà été illustrée notamment dans l'exemple 5 du document WO 2014/037674.

### Exemple 2 - croissance in situ des bactéries et visualisation par fluorescence

Dans cet exemple, on utilise des salmonelles fluorescentes (marquées à la protéine fluorescente verte ou GFP).

On reprend le même protocole que dans l'exemple 1, mais, après l'étape de capture des bactéries, on fait passer un milieu nutritif dans la zone de capture (milieu LB pour « *lysogeny broth* »). Le débit est réglé à 150 nL/min.

On utilise un chauffage autorégulé, grâce à un système composé d'une lame de verre recouverte d'une couche d'oxyde d'indium-étain, d'un thermocouple et d'une alimentation de puissance asservie par une régulation de type PID. Deux électrodes de cuivre collées à la couche d'oxyde et liées au système d'alimentation font circuler un courant, qui induit un échauffement de la lamelle par effet Joule. La température est réglée à la consigne de 37°C.

Lors de la phase de culture, à intervalles réguliers, on coupe l'alimentation en milieu de croissance, afin de passer du régime du lit fluidisé à celui du lit compact, puis on retourne en mode fluidisé après la mesure.

Cela permet de mesurer à intervalles réguliers l'intensité moyenne de la fluorescence du lit compact de particules, par acquisition d'images prises par microscope.

La **figure 4** représente l'évolution de la fluorescence au cours du temps. Celle-ci traduit une forte augmentation du nombre de bactéries au cours de l'étape de croissance.

### Exemple 3 - croissance in situ des bactéries et visualisation par imagerie optique

On reprend le même protocole que dans l'exemple 2, mais sans effectuer de mesure de fluorescence.

Grâce à une caméra, on relève des images du lit (en mode compact). Au fur et à mesure de la division des bactéries, il est possible de visualiser le volume global du lit en mode compact, ou l'avancée du front du lit (c'est-à-dire la dimension longitudinale du lit dans la chambre comprenant la zone de capture).

Le logiciel image J est utilisé pour mesurer l'avancée du front du lit en mode compact au cours du temps.

On constate que, pendant une première phase, le lit de particules ne change pas de taille. Puis, la taille du lit se met à augmenter rapidement, ce qui se traduit par une avancée du front mesuré. Si la croissance des bactéries est de type exponentiel, le mouvement d'avancée du front est approximativement linéaire, étant donné que la largeur de la chambre croît le long de son axe.

En utilisant plusieurs concentrations de départ en salmonelles dans l'échantillon, on constate que l'expansion du volume de lit de particules a un profil similaire, mais que l'instant de début de l'expansion du lit varie. Il est donc possible de déduire la concentration initiale en bactéries à partir de l'instant de début de l'expansion du lit, ou en déterminant le temps au bout duquel cette concentration atteint un certain seuil. Par exemple, pour un modèle simple de croissance exponentielle, le temps nécessaire à l'expansion du lit, t, suit une loi logarithmique en fonction du nombre initial de bactéries N, de forme t = -a ln (N) + b.

Ainsi, en faisant référence à la **figure 5****,** la progression du front du lit de particules dans le canal est comparée pour un échantillon de 50 µL contenant soit 400 bactéries (courbe A), soit 100 bactéries (courbe B), soit 30 bactéries (courbe C), soit 5 bactéries (courbe D). A partir de ces courbes, on peut déduire dans ces conditions expérimentales les paramètres a∼45 et b∼510

### Exemple 4 - procédé comportant une étape d'amplification moléculaire d'ADN par PCR, après l'étape de croissance

Après avoir capturé les bactéries sur les particules magnétiques comme décrit ci-dessus, celles-sont lysées par injection au sein du lit de particules magnétiques d'une solution (10 µL) permettant la lyse et le relargage d'ADN et qui est directement compatible avec une amplification par qPCR (Lyse and Go PCR reagent, Thermo). Afin d'améliorer les performances de la lyse bactérienne et de désactiver les exonucléases, l'ensemble du système subit le cycle de température présenté dans le tableau ci-dessous. La solution récupérée après cette étape de lyse est directement mélangée avec un mélange (ou mix) commercial pour PCR (par exemple iQ-Check Salmonella II Kit) et est ainsi effective pour l'amplification de l'ADN génomique ou plasmidique. Pour ce faire, le lysat est placé dans un thermocycleur pour réaliser la PCR, en ajoutant dans le tube les amorces, et le mix de PCR dans un rapport lysat/mix (1:10, v/v). La présence de salmonelles dans l'échantillon initial est vérifiée par la comparaison du « Ct » fourni par le logiciel d'analyse des résultats de PCR : un Ct inférieur à 30 est choisi comme indicateur d'une présence de salmonelles.

| Cycle | Température (°C) | Temps (secondes) |
|---|---|---|
| 1 | 65 | 30 |
| 2 | 8 | 30 |
| 3 | 65 | 90 |
| 4 | 97 | 180 |
| 5 | 8 | 60 |
| 6 | 65 | 180 |
| 7 | 97 | 60 |
| 8 | 65 | 60 |
| 9 | 80 | maintien |

### Exemple 5 - procédé comportant une étape de relargage d'organismes produits dans le lit fluidisé

Après avoir capturé les bactéries sur les particules magnétiques comme décrit ci-dessus, on collecte au cours du temps en sortie du lit fluidisé le liquide sortant, sous forme d'aliquots de 10 µL, à un rythme d'un aliquot par heure. La quantité de bactéries contenue dans chacun de ces aliquots est ensuite mesurée de façon classique par étalement sur des plaques de culture. Le tableau ci-dessous donne le nombre de bactéries relarguées au cours du temps, à partir d'une quantité initiale de 500 bactéries dans 50 µL, soit une concentration initiale de 100 bactéries dans 10 µL.

| Durée (heures) | Nombre de bactéries dans 10 µL |
|---|---|
| 1 | 3 |
| 2 | 3 |
| 3 | 6 |
| 4 | 50 |
| 5 | 512 |
| 6 | 8488 |

Cette expérience montre d'une part la grande efficacité de capture de l'invention, puisqu'au départ seules 3 bactéries sont relarguées dans 10 µL (contre 100 à l'entrée du dispositif), et également la capacité de l'invention à produire en continu et de façon efficace un grand nombre de bactéries, à une concentration très supérieure à la concentration de l'échantillon initiale.

## Revendications

1. Procédé de détection d'organismes dans un échantillon liquide, comprenant la fourniture d'une zone de capture comportant des particules en milieu liquide et soumise à un flux hydrodynamique, les organismes à détecter étant susceptibles de se lier à ces particules, le procédé comprenant les étapes :
(a) de circulation de l'échantillon à travers la zone de capture ;
(b) de circulation d'un milieu de croissance à travers la zone de capture ; et
(c) de détermination de la présence, de la nature ou de la concentration des organismes dans la zone de capture ;
lesdites particules étant retenues dans la zone de capture sous la forme d'un lit fluidisé par application d'une force s'opposant au flux hydrodynamique pendant au moins une partie de ces étapes.

2. Procédé selon la revendication 1, dans lequel le milieu de croissance comprend des nutriments pour les organismes.

3. Procédé selon l'une des revendications 1 à 2, dans lequel la vitesse du flux hydrodynamique est décroissante dans la zone de capture, dans la direction du flux hydrodynamique.

4. Procédé selon l'une des revendications 1 à 3, dans lequel la retenue des particules dans la zone de capture est obtenue par application d'une force magnétique s'opposant au flux hydrodynamique, les particules étant des particules magnétiques, de manière plus particulièrement préférée super-paramagnétiques.

5. Procédé selon l'une des revendications 1 à 4, dans lequel la force s'opposant au flux hydrodynamique est décroissante dans la zone de capture, dans la direction du flux hydrodynamique.

6. Procédé selon l'une des revendications 1 à 5, dans lequel l'étape (c) est effectuée par la détection, la culture ou l'identification d'organismes ou d'espèces transportés hors de de la zone de capture par flux hydrodynamique au cours de l'étape (b).

7. Procédé selon l'une des revendications 1 à 5, dans lequel l'étape (c) est effectuée par la mesure d'une propriété dans la zone de capture ; ou par la mesure d'une propriété hors de la zone de capture.

8. Procédé selon la revendication 7, dans lequel la propriété mesurée est une propriété biologique ou biochimique, telle qu'une affinité, une capacité de prolifération, un phénotype ou une propriété phénotypique, un génotype ou un caractère génétique, une mutation, un taux d'expression, une morphologie ou une capacité de prolifération ; ou est une force, un déplacement, une pression, un débit, une masse, une densité, une porosité, une viscosité, une élasticité, une viscoélasticité, une densité optique, une turbidité, une propriété de texture, une mesure d'intensité ou un coefficient d'absorption de rayonnement ; ou est le volume occupé par les particules dans la zone de capture ou une dimension de ce volume occupé, optionnellement lorsque les particules sont sous la forme d'un lit compact, la concentration d'organismes dans l'échantillon étant de préférence déterminée en fonction du temps nécessaire au cours de l'étape (b) pour que le volume occupé par les particules dans la zone de capture atteigne une valeur seuil.

9. Ensemble pour la détection d'organismes dans un échantillon liquide comprenant :
• un système fluidique de détection d'organismes dans un échantillon liquide comprenant :
■ au moins une chambre comportant au moins une entrée de fluide et une sortie de fluide et comportant une zone de capture ;
■ des moyens de circulation de l'échantillon liquide à travers la zone de capture ;
■ des moyens de circulation d'un milieu de croissance à travers la zone de capture ;
■ des moyens d'application d'un champ de force dans la zone de capture, ledit champ de force étant configuré pour retenir des particules dans la zone de capture sous la forme d'un lit fluidisé lorsque la zone de capture est soumise à un flux hydrodynamique, la force appliquée s'opposant au flux hydrodynamique, les organismes à détecter étant susceptibles de se lier aux particules ;
■ des moyens de détermination de la présence, de la nature ou de la concentration des organismes dans la zone de capture ; et
• le milieu de croissance dans le système fluidique de détection d'organismes.

10. Ensemble selon la revendication 9, dans lequel les moyens de détermination comprennent des moyens de mesure d'une propriété dans la zone de capture ou dans une deuxième chambre en connexion fluidique avec la chambre comprenant la zone de capture, lesdits moyens de mesure étant de préférence des moyens de mesure de force, de déplacement, de pression, de débit, de masse, de densité, de porosité, de viscosité, d'élasticité, de viscoélasticité, de densité optique, de turbidité, de propriété de texture, d'intensité ou de coefficient d'absorption de rayonnement, et plus particulièrement des moyens de mesure du volume occupé par les particules dans la zone de capture.

11. Procédé de préparation d'organismes à partir d'organismes parents présents dans un échantillon liquide, comprenant la fourniture d'une zone de capture comportant des particules en milieu liquide et soumise à un flux hydrodynamique, les organismes parents étant susceptibles de se lier à ces particules, le procédé comprenant les étapes :
(a) de circulation de l'échantillon à travers la zone de capture et de liaison des organismes parents aux particules ;
(b) de circulation d'un milieu de croissance à travers la zone de capture ; et
(c) de collecte, à la sortie de la zone de capture, d'organismes issus de la division des organismes parents ; les particules étant retenues dans la zone de capture sous la forme d'un lit fluidisé par application d'une force s'opposant au flux hydrodynamique pendant au moins une partie de ces étapes.

12. Procédé selon la revendication 11, dans lequel l'étape (c) s'effectue sans libération des organismes parents liés aux particules.

13. Procédé selon l'une des revendications 11 ou 12, dans lequel l'étape (c) et l'étape (b) se superposent ou alternent au moins en partie.

14. Procédé selon l'une des revendications 11 à 13, dans lequel les organismes sont choisis parmi les bactéries, les parasites unicellulaires ou pluricellulaires, les champignons, les cellules eucaryotes et notamment les cellules de mammifères ou les cellules de plantes, et dans lequel, de préférence, les organismes sont choisis parmi les organismes pathogènes, les cellules souches et les cellules cancéreuses ; ou sont choisis parmi les cellules endothéliales, les cellules hématopoïétiques, les cellules épithéliales, les cellules fœtales, les cellules pluripotentes, les cellules totipotentes non humaines, et les cellules souches pluripotentes induites.

15. Procédé selon l'une des revendications 11 à 14, comprenant en outre une étape de circulation d'un milieu comprenant un agent cible pour les organismes à travers la zone de capture, optionnellement après l'étape de circulation du milieu de croissance, et de préférence le procédé étant pour le criblage de médicaments ou biocides et l'agent cible étant un médicament ou un biocide potentiel.

## Patentansprüche

1. Verfahren zum Nachweis von Organismen in einer flüssigen Probe, umfassend Vorsehen einer Erfassungszone, die einem hydrodynamischen Strom ausgesetzte Partikel in einem flüssigen Medium enthält, wobei sich die nachzuweisenden Organismen an diese Partikel binden können, wobei das Verfahren die Schritte umfasst:
(a) Zirkulieren der Probe durch die Erfassungszone;
(b) Zirkulieren eines Nährmediums durch die Erfassungszone und
(c) Ermitteln des Vorhandenseins, der Natur oder der Konzentration der Organismen in der Erfassungszone,
wobei die Partikel durch Anwenden einer dem hydrodynamischen Strom entgegengesetzten Kraft über mindestens einen Teil dieser Schritte als Wirbelschicht in der Erfassungszone zurückbehalten werden.

2. Verfahren nach Anspruch 1, wobei das Nährmedium Nährstoffe für die Organismen umfasst.

3. Verfahren nach einem der Ansprüche 1 - 2, wobei die Geschwindigkeit des hydrodynamischen Stroms in der Erfassungszone in Richtung des hydrodynamischen Stroms abnimmt.

4. Verfahren nach einem der Ansprüche 1 - 3, wobei das Zurückbehalten der Partikel in der Erfassungszone durch Anwenden einer dem hydrodynamischen Strom entgegengesetzten Magnetkraft erfolgt, wobei es sich bei den Partikeln um magnetische Partikel, besonders bevorzugt um superparamagnetische Partikel, handelt.

5. Verfahren nach einem der Ansprüche 1 - 4, wobei die dem hydrodynamischen Strom entgegengesetzte Kraft in der Erfassungszone in Richtung des hydrodynamischen Stroms abnimmt.

6. Verfahren nach einem der Ansprüche 1 - 5, wobei der Schritt (c) durch Nachweisen, Züchten oder Erkennen der im Schritt (b) vom hydrodynamischen Strom aus der Erfassungszone hinaus beförderten Organismen oder Gattungen erfolgt.

7. Verfahren nach einem der Ansprüche 1 - 5, wobei der Schritt (c) durch Messen einer Eigenschaft in der Erfassungszone oder durch Messen einer Eigenschaft außerhalb der Erfassungszone erfolgt.

8. Verfahren nach Anspruch 7, wobei die zu messende Eigenschaft eine biologische oder biochemische Eigenschaft, wie z.B. eine Affinität, eine Vermehrungsfähigkeit, ein Phänotyp oder eine phänotypische Eigenschaft, en Genotyp oder eine genetische Eigenschaft, eine Mutation, eine Expressionsgeschwindigkeit, eine Morphologie oder eine Vermehrungsfähigkeit ist; oder eine Kraft, eine Verdrängung, ein Druck, ein Durchfluss, eine Masse, eine Dichte, eine Porosität, eine Viskosität, eine Elastizität, eine Viskoelastizität, eine optische Dichte, eine Trübung, eine Textureigenschaft, ein Intensitätsmesswert oder ein Strahlungsabsorptionskoeffizient ist; oder das von den Partikeln belegte Volumen in der Erfassungszone oder eine Dimension des belegten Volumens ist, wahlweise wobei die Partikel in Form einer kompakten Schicht vorliegen, wobei die Konzentration von Organismen in der Probe vorzugsweise als Funktion der Zeit bestimmt wird, die im Schritt (b) benötigt wird, damit das von den Partikeln belegte Volumen in der Erfassungszone einen Schwellenwert erreicht.

9. Einrichtung zum Nachweis von Organismen in einer flüssigen Probe, umfassend:
• ein fluidisches System zum Nachweis von Organismen in einer flüssigen Probe, umfassend:
• mindestens eine Kammer, umfassend mindestens einen Fluideinlass und einen Fluidauslass sowie eine Erfassungszone;
• Mittel zum Zirkulieren der Probe durch die Erfassungszone;
• Mittel zum Zirkulieren eines Nährmediums durch die Erfassungszone;
• Mittel zur Anwendung eines Kraftfeldes in der Erfassungszone, wobei das Kraftfeld derart konfiguriert ist, dass es Partikel in der Erfassungszone in Form einer Wirbelschicht zurückbehält, wenn die Erfassungszone einem hydrodynamischen Strom ausgesetzt wird, wobei sich die angewendete Kraft dem hydrodynamischen Strom entgegensetzt, wobei sich die nachzuweisenden Organismen an die Partikel binden können;
• Mittel zum Ermitteln des Vorhandenseins, der Natur oder der Konzentration der Organismen in der Erfassungszone und
• das Nährmedium im fluidischen System zum Nachweis von Organismen.

10. Einrichtung nach Anspruch 9, wobei die Bestimmungsmittel Mittel zum Messen einer Eigenschaft in der Erfassungszone oder in einer zweiten Kammer, die mit der die Erfassungszone umfassenden Kammer fluidisch verbunden ist, umfassen, wobei die Messmittel vorzugsweise Mittel zum Messen von Kraft, Verdrängung, eines Druck, Durchfluss, Masse, Dichte, Porosität, Viskosität, Elastizität, Viskoelastizität, optischer Dichte, Trübung, Textureigenschaften, Intensität oder Strahlungsabsorptionskoeffizient, und insbesondere Mittel zum Messen des in der Erfassungszone von den Partikeln belegten Volumen sind.

11. Verfahren zum Erzeugung von Organismen aus Ausgangsorganismen, die in einer flüssigen Probe vorhanden sind, umfassend Vorsehen einer Erfassungszone, die einem hydrodynamischen Strom ausgesetzte Partikel in einem flüssigen Medium umfasst, wobei sich die Ausgangsorganismen an diese Partikel binden können, wobei das Verfahren die Schritte umfasst:
(a) Zirkulieren der Probe durch die Erfassungszone und Binden der Ausgangsorganismen an die Partikel,
(b) Zirkulieren eines Nährmediums durch die Erfassungszone und
(c) Sammeln von Organismen, die von der Teilung der Ausgangsorganismen herrühren, am Ausgang der Erfassungszone,
wobei die Partikel durch Anwenden einer dem hydrodynamischen Strom entgegengesetzten Kraft über mindestens einen Teil dieser Schritte als Wirbelschicht in der Erfassungszone zurückbehalten werden.

12. Verfahren nach Anspruch 11, wobei der Schritt (c) erfolgt, ohne dass die an die Partikel gebundenen Ausgangsorganismen freigesetzt werden.

13. Verfahren nach einem der Ansprüche 11 oder 12, wobei der Schritt (c) und der Schritt (b) sich mindestens teilweise überlappen oder abwechseln.

14. Verfahren nach einem der Ansprüche 11 - 13, wobei die Organismen aus folgender Gruppe gewählt sind: Bakterien, ein- oder mehrzellige Parasiten, Pilze, eukaryotische Zellen, insbesondere Säuger- oder Pflanzenzellen, und wobei die Organismen vorzugsweise aus folgender Gruppe gewählt sind: Krankheitserreger, Stammzellen und Krebszellen, oder gewählt sind aus Endothelzellen, hämatopoetischen Zellen, Epithelzellen, fötalen Zellen, pluripotenten Zellen, nicht menschlichen totipotenten Zellen und induzierten pluripotenten Stammzellen.

15. Verfahren nach einem der Ansprüche 11 - 14, ferner umfassend einen Schritt des Zirkulierens eines Mediums, das ein Zielagens der Organismen umfasst, durch die Erfassungszone, ggf. im Anschluss an den Schritt des Zirkulierens des Nährmediums, wobei das Verfahren vorzugsweise zum Screening von Arzneimitteln oder Bioziden dient und das Zielagens ein potentielles Arzneimittel oder Biozid ist.

## Claims

1. A method for detecting organisms in a liquid sample, comprising providing a capture area including particles in a liquid medium and subjected to a hydrodynamic flow, wherein the organisms to be detected are capable of binding to these particles, wherein the method comprises the steps of:
(a) circulating the sample through the capture area;
(b) circulating a growth medium through the capture area; and
(c) determining the presence, nature or concentration of the organisms in the capture area;
said particles being retained in the capture area as a fluidized bed by applying a force opposed to the hydrodynamic flow during at least part of these steps.

2. The method according to claim 1, wherein the growth medium comprises nutrients for the organisms.

3. The method according to one of claims 1 to 2, wherein the velocity of the hydrodynamic flow decreases in the capture area in the direction of the hydrodynamic flow.

4. The method according to one of claims 1 to 3, wherein the retention of the particles in the capture area is obtained by applying a magnetic force opposed to the hydrodynamic flow, the particles being magnetic particles, more preferably super-paramagnetic particles.

5. The method according to any one of claims 1 to 4, wherein the force opposed to the hydrodynamic flow decreases in the capture area, in the direction of the hydrodynamic flow.

6. The method according to one of claims 1 to 5, wherein step (c) is carried out by the detection, the culture or the identification of organisms or species transported out of the capture area by a hydrodynamic flow during step (b).

7. The method according to one of claims 1 to 5, wherein step (c) is carried out by measuring a property in the capture area; or by measuring a property outside the capture area.

8. The method according to claim 7, wherein the measured property is a biological or biochemical property, such as an affinity, a proliferation capability, a phenotype or a phenotype property, a genotype or a genetic character, a mutation, an expression level, a morphology or a proliferation capability; or is a force, a displacement, a pressure, a flow rate, a mass, a density, a porosity, a viscosity, an elasticity, a viscoelasticity, an optical density, a turbidity, a texture property, a measurement of intensity or a radiation absorption coefficient; or is the volume occupied by the particles in the capture area or a dimension of this occupied volume, optionally when the particles are in the form of a compact bed, the concentration of organisms in the sample being preferably determined as a function of the time required during step (b) for the volume occupied by the particles in the capture area to reach a threshold value.

9. A set for detecting organisms in a liquid sample comprising:
- a fluidic system for detecting organisms in a liquid sample, comprising:
∘ at least one chamber including at least one fluid inlet and one fluid outlet and including a capture area;
∘ means for circulating the liquid sample through the capture area;
∘ means for circulating a growth medium through the capture area;
∘ means for applying a force field in the capture area, said force field being configured so as to retain particles in the capture area as a fluidized bed when the capture area is subjected to a hydrodynamic flow, wherein the organisms to be detected are capable of binding to the particles;
∘ means for determining the presence, nature or concentration of the organisms in the capture area; and
- the growth medium in the fluidic system for detecting organisms.

10. The set according to one of claims 9, wherein the means for determining comprise means for measuring a property in a second chamber in fluidic connection with the chamber comprising the capture area, said means preferably being means for measuring a force, a displacement, a pressure, a flow rate, a mass, a density, a porosity, a viscosity, an elasticity, a viscoelasticity, an optical density, a turbidity, a texture property, a radiation intensity or absorption coefficient, and more particularly means for measuring the volume occupied by the particles in the capture area.

11. A method for preparing organisms from parent organisms present in a liquid sample, comprising providing a capture area including particles in a liquid medium and subjected to a hydrodynamic flow, wherein the parent organisms are capable of binding to these particles, the method comprising the steps of:
(a) circulating the sample through the capture area and binding the parent organisms to the particles;
(b) circulating a growth medium through the capture area; and
(c) collecting, at the outlet of the capture area, organisms stemming from the division of the parent organisms;
the particles being retained in the capture area in the form of a fluidized bed by applying a force opposed to the hydrodynamic flow during at least part of these steps.

12. The method according to claim 11, wherein step (c) is carried out without releasing the parent organisms bound to the particles.

13. The method according to one of claims 11 or 12, wherein step (c) and step (b) are superimposed or at least partly alternate.

14. The method according to one of claims 11 to 13, wherein the organisms are selected from bacteria, unicellular or pluricellular parasites, fungi, eukaryotic cells and notably mammal cells or plant cells, and wherein, preferably, the organisms are selected from pathogenic organisms, stem cells and cancer cells; or are selected from endothelial cells, hematopoietic cells, epithelial cells, fetal cells, pluripotent cells, non-human totipotent cells, and induced pluripotent stem cells.

15. The method according to one of claims 11 to 14, further comprising a step of circulating a medium comprising a target agent for the organisms through the capture area, optionally after the step of circulating the growth medium, and wherein preferably the method is for screening drugs or biocides and the target agent is a potential drug or biocide.
